(19) Europäisches Patentamt
European Patent Office
Office européen des brevets

(11) Publication number : 0 473 542 B1

(12) # EUROPEAN PATENT SPECIFICATION

(45) Date of publication of patent specification : **11.05.94 Bulletin 94/19**

(51) Int. Cl.$^5$ : **C07C 323/25,** C07D 215/06, C08K 5/37, C10M 135/22

(21) Application number : **91810627.9**

(22) Date of filing : **09.08.91**

(54) **Thioaminal stabilizers.**

(30) Priority : **16.08.90 US 568379**

(43) Date of publication of application : **04.03.92 Bulletin 92/10**

(45) Publication of the grant of the patent : **11.05.94 Bulletin 94/19**

(84) Designated Contracting States : **DE FR GB IT**

(56) References cited :
**EP-A- 0 119 160**
**US-A- 3 399 041**
**US-A- 4 402 842**
**US-A- 4 633 019**
**CHEMICAL ABSTRACTS, vol. 112, no. 7, 12 February 1990, Columbus, Ohio, US; abstract no. 54456C, page 648**

(73) Proprietor : **CIBA-GEIGY AG**
**Klybeckstrasse 141**
**CH-4002 Basel (CH)**

(72) Inventor : **Steinberg, David H.**
**2216 Wilson Avenue**
**Bronx, NY 10469 (US)**
Inventor : **Luzzi, John**
**Ann Road - RFD No.1**
**Carmel, NY 10512 (US)**
Inventor : **Evans, Samuel, Dr.**
**Route des Charbonnières 17**
**CH-1723 Marly (CH)**
Inventor : **Cortolano, Frank**
**3 East Maple Street**
**Valhalla, NY 10595 (US)**

EP 0 473 542 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Field of the Invention

This invention relates to novel thioaminals. It also relates to compositions of organic materials which are stabilized against oxidative degradation by the presence therein of a broader range of thioaminal compounds.

### Background of the Invention

It is known that organic materials can be stabilized by the addition of antioxidants such as compounds with amine and/or sulfide functionality in order to avoid degradation, decomposition, sludge formation, viscosity increases, loss of elasticity, embrittlement and the like. US Patent No. 3,535,243,for example, discloses the use of diaminonaphthalenes as antioxidants for ester lubricants. US Patent No. 3,536,706 discloses phenothiazines for similar purposes. US Patent No. 4,785,095 discloses N-substituted phenothiazines as anti oxidants for lubricating oils. US Patent 2,281,521 discloses antioxidants which are reaction products of mercaptans, formaldehyde and secondary or tertiary aromatic amines as shown below.

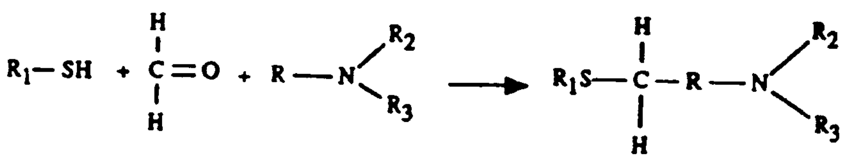

US Patent No. 4,402,842 relates to a product by process wherein the product is made by reacting an alkyl, alkenyl, cycloalkyl or cycloalkenyl mono- or diamine with certain mercaptans and aldehydes and the use thereof as friction reducing additives. US 2,703,784 discloses the use of corrosion inhibitors containing a product made from an aldehyde, a mercaptan and a dimercaptan. US 2,786,616 discloses lubricant compositions containing products derived from formaldehyde, aliphatic amines and an alkylthiohydroquinone. US 2,971,909 discloses a non-corrosive lubricant composition containing the reaction product of 2-amino-5-thio-1,3,4-dithiazolidine, an aldehyde and a mercaptan. US 3,399,041 discloses a method of stabilizing lubricating oils by the addition thereto of a diaminodithiaalkane. The instant thioaminals are not specifically disclosed in any of the above publications.

Various thioaminals derived from diphenylamine, arylthiols or silylthiols and formaldehyde are disclosed in Azerb. Khim. Zh., (4), 13-15 (CA 72:78567), Zh. Org. Khim., 5(10), 1813-15 (CA 72:21458), J. Organomet. Chem., 97(2), 159-65 and Uch. Zap. Azerb. Um-T. Ser. Khim. N., (3), 26-9 (Ca 84:59020), Zh. Obshch. Khim., 43(7), 1540-2 (Ca 80:14996). The instant alkylthioaminal derivatives are neither taught nor suggested. US Patent No. 4,415,591 discloses thioaminals as antihypertensives for animals.

### Summary of the Invention

It has now been determined that certain thioaminal compounds show surprising effectiveness in stabilizing organic materials which are subject to oxidative and thermal degradation.

Thus, it is the primary object of this invention to provide novel compositions of organic materials which are stabilized against oxidative and thermal degradation by the incorporation therein of thioaminal compounds of formulae A-G.

It is a further object to provide novel compounds of formulae I-VIII.

Various other objects and advantages of the invention will become evident from the following description thereof.

### Detailed Disclosure

The invention pertains to compositions stabilized against degradation which comprise

(a) an organic substrate subject to oxidative, thermal or light induced degradation and

(b) as stabilizer, an effective stabilizing amount of a compound of formulae A- H :

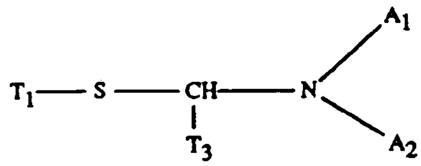

(A)

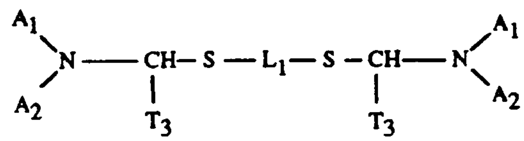

(B)

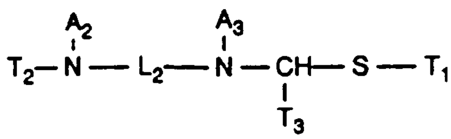

(C)

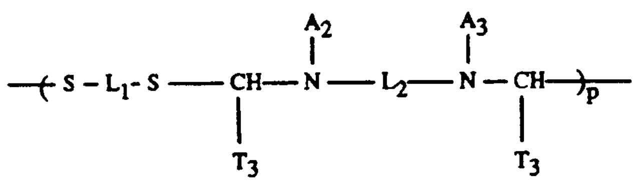

(D)

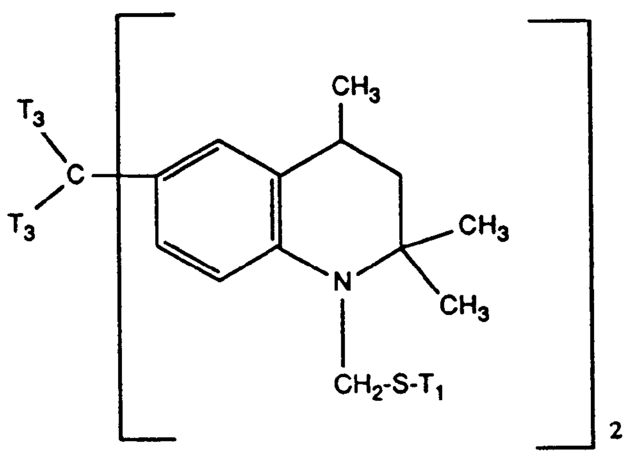

(E)

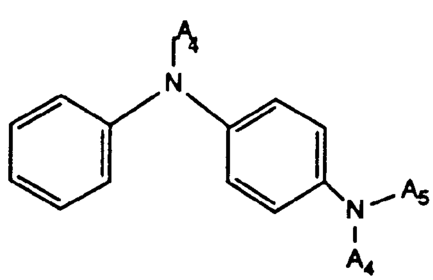

(F)

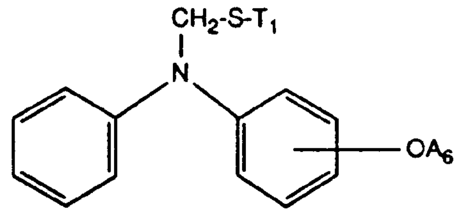

(G)

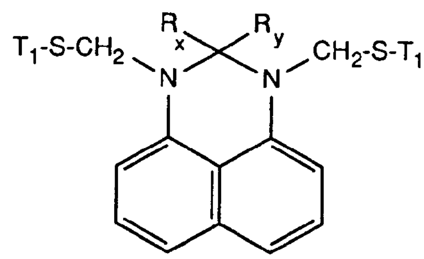

(H)

wherein

$T_1$ is $C_6$-$C_{12}$aryl, $C_1$-$C_{30}$alkyl, $C_5$-$C_{12}$cycloalkyl or $-CH_2CH_2OH$;

$T_2$ is

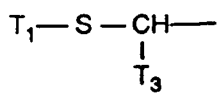

or H when $A_2$ is phenyl;

$T_3$ is H, $C_1$-$C_{14}$alkyl, phenyl or phenyl-$C_1$-$C_4$alkyl;

$A_1$, $A_2$ and $A_3$ independently of each other, are H, $C_1$-$C_{18}$alkyl,

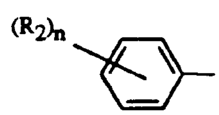

,

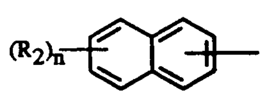

,

or $-CH(T_3)-S-T_1$; or

$A_1$ and $A_2$ together with the nitrogen atom to which they are attached are

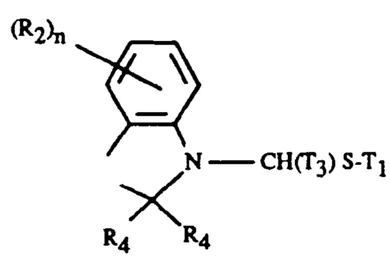

,

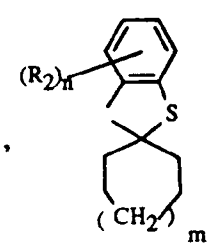

,

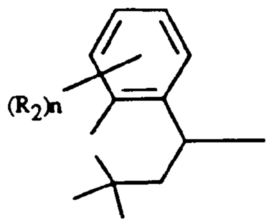 . 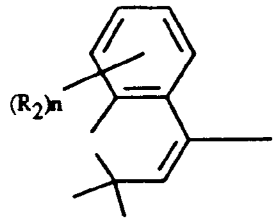 . or 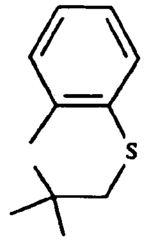 ;

$R_2$ is H, $C_1$-$C_{30}$alkyl or -$C(R_6)(R_7)$phenyl;
$R_4$ is $C_1$-$C_{16}$alkyl or two $R_4$ groups together are pentamethylene;
$R_6$ and $R_7$ are independently H or phenyl;
$R_x$ and $R_y$ are independently H, $C_1$-$C_{18}$alkyl, $C_5$-$C_{12}$cycloalkyl or phenyl;
$A_4$ independently is H or -$CH_2$-S-$T_1$, at least one of $A_4$ being -$CH_2$-S-$T_1$;
$A_5$ is H, $C_1$-$C_{16}$alkyl, $C_6$-$C_{12}$aryl or -$CH_2$-S-$T_1$;
$A_6$ is H or $C_1$-$C_{25}$alkyl;
m is 1 or 2;
n is 1, 2, 3 or 4;
p is 1 to 20;
$L_1$ is a direct bond, linear or branched $C_2$-$C_{12}$alkylene, $C_6$-$C_{12}$arylene or

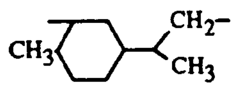 ;

and
$L_2$ is $C_2$-$C_{12}$alkylene, $C_6$-$C_{12}$arylene, xylylene, $C_4$-$C_{12}$alkylene which is interrupted by -N(CH($T_3$)-S-$T_1$) or $L_2$ is

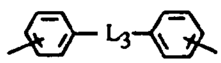

wherein $L_3$ is a direct bond, $C_1$-$C_6$alkylene, S, -NH- or O.

Representative $T_1$, $T_2$, $T_3$, $A_1$-$A_3$ and $L_1$-$L_3$ groups are as follows.

When $T_1$ is $C_1$-$C_{30}$alkyl, $T_1$ is for example methyl, pentyl, octyl, decyl, octadecyl eicosyl, or pentaeicosyl. Preferably $T_1$ is $C_8$-$C_{12}$alkyl, especially octyl, tert-nonyl, or tert.dodecyl, or -$CH_2CH_2OH$. When $T_1$ is $C_6$-$C_{12}$aryl, it is preferably phenyl. $R_2$ is preferably tert.-butyl or tert.-octyl. $T_3$ is preferably H, methyl, butyl, hexyl, phenyl, tolyl or xylyl and most preferably H. $A_1$ and $A_3$ are preferably phenyl, naphthyl, tolyl or xylyl. Another preferred embodiment of $A_2$ is -CH($T_3$)-S-$T_1$. $A_3$ is preferably -CH($T_3$)-S-$T_1$, $C_1$-$C_{12}$alkyl or phenyl. $L_1$ is preferably $C_2$-$C_6$alkylene and most preferably $C_6$alkylene. $L_2$ is preferably phenylene or

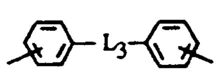

wherein $L_3$ is $CH_2$. $T_2$ is preferably -CH($T_3$)-S-$T_1$ or H with $A_2$ being phenyl.

This invention also pertain to novel compounds. The stabilizing compounds utilized in the compositions of this invention correspond to formulae I to VIII:

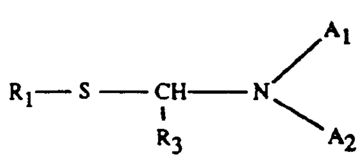 (I)

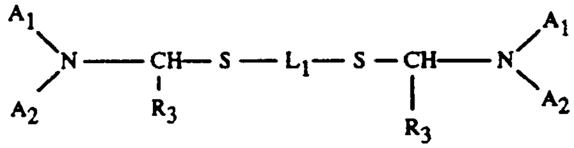

(II)

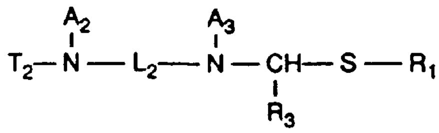

(III)

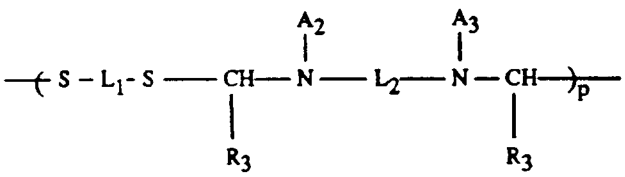

(IV)

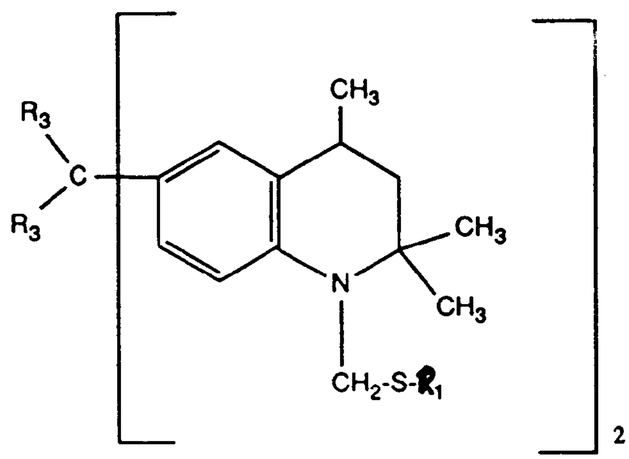

(V)

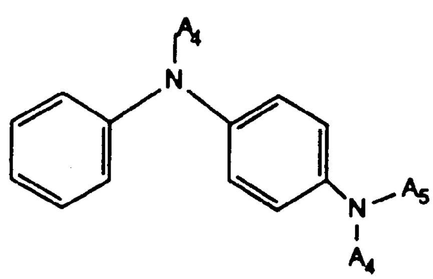

(VI)

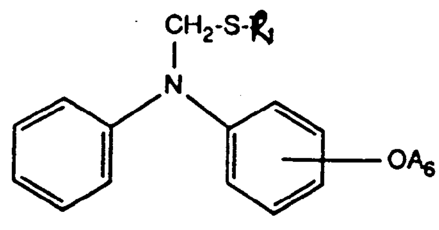

(VII)

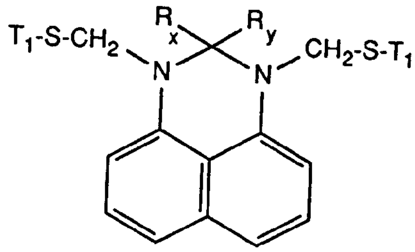

(VIII)

wherein

$T_1$, $R_x$ and $R_y$ are as defined in formula (H),

$R_1$ is $C_1$-$C_{30}$alkyl, $C_5$-$C_{12}$-cycloalkyl, phenyl or $-CH_2CH_2OH$;

$T_2$ is

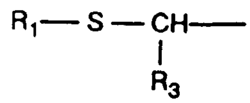

or H when $A_2$ is phenyl;

$R_3$ is H, $C_1$-$C_{14}$alkyl, phenyl or phenyl-$C_1$-$C_4$alkyl;

$R_4$ is $C_1$-$C_{16}$alkyl or two $R_4$ groups together are pentamethylene;

$A_1$, $A_2$ and $A_3$, independently of each other, are H, $C_1$-$C_{18}$alkyl,

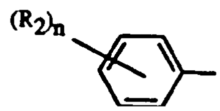
,

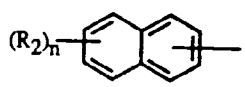
,

or $-CH(R_3)-S-R_5$ wherein $R_2$ is H, $C_1$-$C_{30}$alkyl or $-C(R_5)(R_7)$phenyl, and $R_5$ is $C_6$-$C_{12}$aryl, $C_1$-$C_{30}$alkyl, $C_5$-$C_{12}$cycloalkyl or $-CH_2CH_2OH$; or

$A_1$ and $A_2$ together with the nitrogen atom to which they are attached are

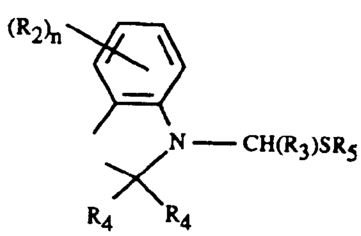

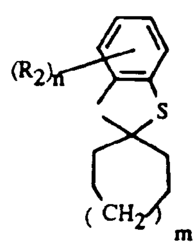
,

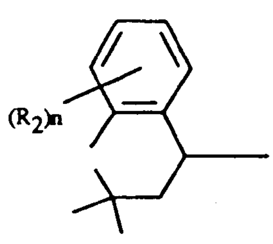
,
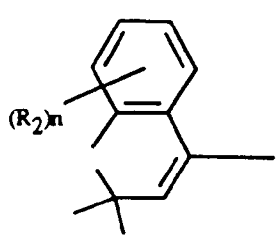
, or
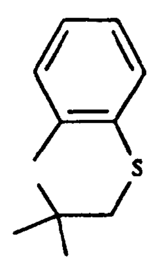
;

$A_4$ independently is H or $-CH_2-S-R_1$, at least one of $A_4$ being $-CH_2-S-R_1$;
$A_5$ is H, $C_1$-$C_{16}$alkyl, $C_6$-$C_{12}$aryl or $-CH_2-S-R_1$;
$A_6$ is H or $C_1$-$C_{25}$alkyl;
m is 0, 1 or 2;
n is 1, 2, 3 or 4;
p is 1 to 20;
$L_1$ is a direct bond, linear or branched $C_2$-$C_{12}$alkylene, $C_6$-$C_{12}$arylene, or

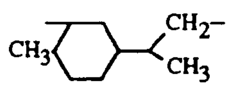
;

and
$L_2$ is $C_2$-$C_{12}$alkylene, $C_6$-$C_{12}$arylene, xylylene, $C_4$-$C_{12}$alkylene which is interrupted by $-N(CH(R_3)-S-R_5)$ wherein $R_5$ is as defined above or $L_2$ is

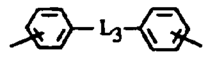

wherein $L_3$ is a direct bond, $C_1$-$C_6$alkylene, S, -NH- or O.

Representative $R_1$-$R_4$, $A_1$-$A_3$ and $L_1$-$L_3$ groups are as follows.

When $R_1$ is $C_1$-$C_{30}$alkyl, $R_1$ is for example methyl, pentyl, decyl, octadecyl, eicosyl, and pentaeicosyl. Preferably $R_1$ is $C_8$-$C_{12}$alkyl, especially octyl, tert.nonyl or tert.dodecyl or $-CH_2CH_2OH$. $R_2$ is preferably tert.-butyl or tert.-octyl.
$R_3$ is preferably H, methyl, butyl or hexyl and most preferably H.
$R_4$ is preferably pentamethylene.
$A_1$ or $A_2$ are preferably phenyl, naphthyl, tolyl or xylyl.
Another preferred embodiment of $A_2$ is $-CH(R_3)-S-R_5$. $A_3$ is preferably $-CH(R_3)-S-R_5$, $C_1$-$C_{12}$alkyl or phenyl.
$L_1$ is preferably $C_2$-$C_6$alkylene and most preferably $C_6$alkylene.
$L_2$ is preferably phenylene or

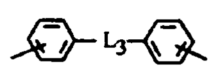

wherein $L_3$ is $-CH_2-$.

The thioaminal compounds can be prepared by a condensation reaction using the requisite amount of the appropriately substituted thiol, aldehyde and amine. Thus, compounds of formula A and I are prepared from the appropriately substituted monothiol, aldehyde and monoamine. Compounds of formula B and II are correspondingly prepared from a dithiol, an aldehyde and a monoamine. Compounds of formula C, F, II and VI are prepared form a monothiol, an aldehyde and a diamine. The polymeric or oligomeric compounds of formula D and IV are made from a dithiol, an aldehyde and a diamine.

Compounds of formula G can be synthesized by reacting the hydroxy or alkoxy diphenylamine with formaldehyde and a monothiol in the presence or absence of a solvent, e.g. ethanol, isopropanol or the like.

Compounds of formula E are prepared by reacting together a mixture of 2 moles of a monothiol, 2 moles of formaldehyde and 1 mole of the corresponding bis-tetrahydroquinoline compound (formula E, unsubstituted at the N atom).

Similarly, compounds of formula F can be obtained by treatment of the corresponding 4-amino or 4-N-alkyl(aryl)amino diphenylamine with the necessary amount of monothiol and aldehyde.

In a similar fashion compounds of formula H can be prepared by reacting together a mixture of 2 moles of a monothiol, 2 moles of formaldehyde and 1 mole of the corresponding bis-amine naphthalene product.

The compounds utilized in the present invention are particularly effective in stabilizing organic materials subject to oxidative or thermal degradation such as lubricants, elastomers and other polymers. Substrates in which these compounds are particularly useful are lubricants and elastomers. These preferred substrates may be defined as:

1. Lubricant which may be an oil or a grease based on mineral or synthetic fluids. These lubricants are well known to those skilled in the art. The term mineral oil includes all mineral oils used for lubricant purposes, such as hydrocarbon mineral oils. The synthetic fluid may be, for example, an aliphatic or aromatic carboxylic ester or polymeric ester, a polyalkylene oxide, a phosphoric acid ester, polyalpha-olefins, or a silicone. Greases may be obtainable from these by adding metal soaps or other thickeners.
2. Natural or synthetic rubber, e.g. natural latex or styrene/butadiene copolymers.
In general polymers which can be stabilized include
1. Polymers of monoolefins and diolefins, for example polyethylene (which optionally can be crosslinked), polypropylene, polyisobutylene, polybutene-1, polymethylpentene-1, polyisoprene or polybutadiene, as well as polymers of cycloolefins, for instance of cyclopenteneor norbornene.
2. Mixtures of the polymers mentioned under 1), for example mixtures of polypropylene with polyisobutylene.
3. Copolymers of monoolefins and diolefins with each other or with other vinyl monomers, such as, for example, ethylene/propylene, propylene/butene-1, propylene/isobutylene, ethylene/butene-1, propylene/butadiene, isobutylene/isoprene, ethylene/alkyl acrylates, ethylene/alkyl methacrylates, ethylene/vinyl acetate or ethylene/acrylic acid copolymers and their salts (ionomers) and terpolymers of ethylene with propylene and a diene, such as hexadiene, dicyclopentadiene or ethylidene-norbornene.
4. Polystyrene, poly-(p-methylstyrene).
5. Copolymers of styrene or methylstyrene with dienes or acrylic derivatives, such as, for example, styrene/butadiene, styrene/acrylonitrile, styrene/ethyl methacrylate, styrene/butadiene/ethyl accrylate, styrene/acrylonitrile/methyl acrylate; mixtures of high impact strength from styrene copolymers and another polymer, such as, for example, from a polyacrylate, a diene polymer or an ethylene/propylene/diene terpolymer; and block polymers of styrene, such as, for example, styrene/butadiene/styrene, styrene/isoprene/styrene, styrene/ethylene/butylene/styrene or styrene/ethylene/propylene/styrene.
6. Graft copolymers of styrene, such as, for example, styrene on polybutadiene, styrene and acrylonitrile on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS-, MBS-, ASA- or AES-pol ymers.
7. Halogen-containing polymers, such as polychloroprene, chlorinated rubbers, chlorinated or sulfochlorinated polyethylene, epichlorohydrin homo- and copolymers, polymers from halogen-containing vinyl compounds, as for example, polyvinylchloride, polyvinylidene chloride, polyvinyl fluoride, polyvinylidene fluoride, as well as copolymers thereof, as for example, vinyl chloride/vinylidene chloride, vinyl chloride/vinyl acetate, vinylidene chloride/vinyl acetate copolymers, or vinyl fluoride/vinyl ether copolymers.
8. Polymers which are derived from $\alpha,\beta$-unsaturated acids and derivatives thereof, such as polyacrylates and polymethacrylates, polyacrylamide and polyacrylonitrile.
9. Copolymers from the monomers mentioned under 8) with each other or with other unsaturated monomers, such as, for instance, acrylonitrile/butadiene, acrylonitrile/alkyl accrylate, acrylonitrile/alkoxyalkyl acrylate or acrylonitrile/vinyl halogenide copolymers or acrylonitrile/alkyl methacrylate/butadiene terpolymers.
10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinylbutyral, polyallyl phthalate or polyallyl-melamine.
11. Homopolymers and copolymers of cyclic ethers, such as polyalkylene glycols, polyethylene oxide, polypropylene oxide or copolymers thereof with bis-glycidyl ethers.
12. Polyacetals, such as polyoxymethylene and those polyoxymethylenes which contain ethylene oxide as comonomer.
13. Polyphenylene oxides and sulfides, and mixtures of polyphenylene oxides with polystyrene.
14. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).
15. Polyamides and copolyamides which are derived from diamines and dicarboxylic acids and/or from aminocarboxylic acids or the corresponding lactams, such as polyamide 4, polyamide 6, polyamide 6/6, polyamide 6/10, polyamide 11, polyamide 12, poly-2,4,4-trimethylhexamethylene terephthalamide, poly-p-phenylene terephthalamide or poly-m-phenylene isophthalamide, as well as copolymers thereof with polyethers, such as for instance with polyethylene glycol, polypropylene glycol or polytetramethylene glycols.
16. Polyureas, polyimides and polyamide-imides.
17. Polyesters which are derived from dicarboxylic acids and diols and/or from hydroxycarboxylic acids

or the corresponding lactones, such as polyethylene terephthalate, polybutylene terephthalate, poly-1,4-dimethylol-cyclohexane terephthalate, poly-[2,2-(4-hydroxyphenyl)-propane] terephthalate and polyhydroxybenzoates as well as block-copolyether-esters derived from polyethers having hydroxyl end groups.

18. Polycarbonates.

19. Polysulfones, polyethersulfones and polyetherketones.

20. Crosslinked polymers which are derived from aldehydes on the one hand and phenols, ureas and melamines on the other hand, such as phenol/formaldehyde resins, urea/formaldehyde resins and melamine/formaldehyde resins.

21. Drying and non-drying alkyd resins.

22. Unsaturated polyester resins which are derived from copolyesters of saturated and unsaturated dicarboxylic acids with polyhydric alcohols and vinyl compounds as crosslinking agents, and also halogen-containing modifications thereof of low flammability.

23. Thermosetting acrylic resins, derived from substituted acrylic esters, such as epoxy-acrylates, urethane-acrylates or polyester acrylates.

24. Alkyd resins, polyester resins or acrylate resins in admixture with melamine resins, urea resins, polyisocyanates or epoxide resins as crosslinking agents.

25. Crosslinked epoxide resins which are derived from polyepoxides, for example from bis-glycidyl ethers or from cycloaliphatic diepoxides.

26. Natural polymers, such as cellulose, rubber, gelatin and derivatives thereof which are chemically modified in a polymer homologous manner, such as cellulose acetates, cellulose propionates and cellulose butyrates, or the cellulose ethers, such as methyl cellulose.

27. Mixtures of polymers as mentioned above, for example PP/EPDM, Polyamide 6/EPDM or ABS, PVC/EVA, PVC/ABS, PVC/MBS, PC/ABS, PBTP/ABS.

28. Naturally occuring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellitates) and also mixtures of synthetic esters with mineral oils in any weight ratios, which materials may be used as plasticizers for polymers or as textile spinning oils, as well as aqueous emulsions of such materials.

29. Aqueous emulsions of natural or synthetic rubber, e.g. natural latex or latices of carboxylated styrene/butadiene copolymers.

30. Polysiloxanes such as the soft, hydrophilic polysiloxanes described for example, in U.S. Patent No. 4,259,467; and the hard polyorganosiloxanes described, for example, in U.S. Patent No. 4,355,147.

31. Polyketimines in combination with unsaturated acrylic polyacetoacetate resins or with unsaturated acrylic resins. The unsaturated acrylic resins include the urethane acrylates, polyether acrylates, vinyl or acryl copolymers with pendant unsaturated groups and the acrylated melamines. The polyketimines are prepared from polyamines and ketones in the presence of an acid catalyst.

32. Radiation curable compositions containing ethylenically unsaturated monomers or oligomers and a polyunsaturated aliphatic oligomer.

33. Epoxymelamine resins such as light-stable epoxy resins crosslinked by an epoxy functional coetherified high solids melamine resin such as LSE-4103 (Monsanto).

In general, the compounds of the present invention are employed in from about 0.01 to about 5% by weight of the stabilized composition, although this will vary with the particular substrate and application. An advantageous range is from about 0.5 to about 2%, and especially 0.1 to about 1%.

The stabilizers of the instant invention may readily be incorporated into the organic polymers by conventional techniques, at any convenient stage prior to the manufacture of shaped articles therefrom. For example, the stabilizer may be mixed with the polymer in dry powder form, or a suspension or emulsion of the stabilizer may be mixed with a solution, suspension, or emulsion of the polymer. The resulting stabilized polymer compositions of the invention may optionally also contain various conventional additives, such as the following.

1. Antioxidants

1.1. Alkylated monophenols, for example,

2,6- di- tert- butyl- 4- methylphenol
2- tert.butyl-4,6-dimethylphenol
2,6-di-tert-butyl-4-ethylphenol
2,6-di-tert-butyl-4-n-butylphenol
2,6-di-tert-butyl-4-i-butylphenol
2,6-di-cyclopentyl-4-methylphenol
2-($\alpha$-methylcyclohexyl)-4,6-dimethylphenol
2,6-di-octadecyl-4-methylphenol

2,4,6-tri-cyclohexylphenol
2,6-di-tert-butyl-4-methoxymethylphenol
1.2. Alkylated hydroquinones, for example,
2,6-di-tert-butyl-4-methoxyphenol
2,5-di-tert-butyl-hydroquinone
2,5-di-tert-amyl-hydroquinone
2,6-diphenyl-4-octadecyloxyphenol
1.3. Hydroxylated thiodiphenyl ethers, for example,
2,2'-thio-bis-(6-tert-butyl-4-methylphenol)
2,2'-thio-bis-(4-octylphenol)
4,4'-thio-bis-(6-tert-butyl-3-methylphenol)
4,4'-thio-bis-(6-tert-butyl-2-methylphenol)
1.4. Alkylidene-bisphenols, for example,
2,2'-methylene-bis-(6-tert-butyl-4-methylphenol)
2,2'-methylene-bis-(6-tert-butyl-4-ethylphenol)
2,2'-methylene-bis-[4-methyl-6-(α-methylcyclohexyl)-phenol]
2,2'-methylene-bis-(4-methyl-cyclohexylphenol)
2,2'-methylene-bis-(6-nonyl-4-methylphenol)
2,2'-methylene-bis-[6-(α-methylbenzyl)-4-nonylphenol]
2,2'-methylene-bis-[6-(α,α-dimethylbenzyl)4-nonylphenol]
2,2'-methylene-bis-(4,6-di-tert-butylphenol)
2,2'-ethylidene-bis-(4,6-di-tert-butylphenol)
2,2'-ethylidene-bis-(6-tert-butyl-4-isobutylphenol)
4,4'-methylene-bis-(2,6-di-tert-butylphenol)
4,4'-methylene-bis-(6-tert-butyl-2-methylphenol)
1,1-bis-(5-tert-butyl-4-hydroxy-2-methylphenyl-butane
2,6-di-(3-tert-butyl-5-methyl-2-hydroxybenzyl)-4-methylphenol
1,1,3-tris-(5-tert-butyl-4-hydroxy-2-methylphenyl)-butane
1,1-bis-(5-tert-butyl-4-hydroxy-2-methylphenyl)-3-n-dodecylmerca ptobutane ethyleneglycol bis-[3,3-bis-(3'-tert-butyl-4'-hydroxyphenyl)butyrate]
di-(3-tert-butyl-4-hydroxy-5-methylphenyl)-dicyclopentadiene
di-[2-(3'-tert-butyl-2'-hydroxy-5'-methyl-benzyl)-6-tert-butyl-4-methylphenyl] terephthalate.
1.5. Benzyl compounds, for example,
1,3,5-tri-(3,5-di-tert-butyl-4-hydroxybenzyl)-2,4,6-trimethylben zene di-(3,5-di-tert-butyl-4-hydroxybenzyl) sulfide
3,5-di-tert-butyl-4-hydroxybenzyl-mercapto-acetic acid isooctyl ester bis-(4-tert-butyl-3-hydroxy-2,6-dimethylbenzyl)dithiol terephthalate
1,3,5-tris-(3,5-di-tert-butyl-4-hydroxybenzyl) isocyanurate
1,3,5-tris-(4-tert-butyl-3-hydroxy-2,6-dimethylbenzyl) isocyanurate
3,5-di-tert-butyl-4-hydroxybenzyl-phosphoric acid dioctadecyl ester
3,5-di-tert-butyl-4-hydroxybenzyl-phosphoric acid monoethyl ester, calcium-salt
1.6. Acylaminophenols, for example,
4-hydroxy-lauric acid anilide
4-hydroxy-stearic acid anilide
2,4-bis-octylmercapto-6-(3,5-tert-butyl-4-hydroxyanilino)-s-tria zine octyl-N-(3,5-di-tert-butyl-4-hydroxyphenyl)-carbamate
1.7. Esters of β-(3,5-di-tert-butyl-4-hydroxyphenyl)-propionic acid with monohydric or polyhydric alcohols, for example,

| | |
|---|---|
| methanol | diethylene glycol |
| octadecanol | triethylene glycol |
| 1,6-hexanediol | pentaerythritol |
| neopentyl glycol | tris-hydroxyethyl isocyanurate |
| thiodiethylene glycol | di-hydroxyethyl oxalic acid diamide |

1.8. Esters of β-(5-tert-butyl-4-hydroxy-3-methylphenyl)-propionic acid with monohydric or polyhydric alcohols, for example,

| | |
|---|---|
| methanol | diethylene glycol |
| octadecanol | triethylene glycol |
| 1,6-hexanediol | pentaerythritol |
| neopentyl glycol | tris-hydroxyethyl isocyanurate |
| thiodiethylene glycol | di-hydroxyethyl oxalic acid diamide |

1.9. Amides of β-(3,5-di-tert-butyl-4-hydroxyphenyl)-propionic acid for example,
N,N'-di-(3,5-di-tert-butyl-4-hydroxyphenylpropionyl)-hexamethyle nediamine
N,N'-di-(3,5-di-tert-butyl-4-hydroxyphenylpropionyl)-trimethylen ediamine
N,N'-di-(3,5-di-tert-butyl-4-hydroxyphenylpropionyl)-hydrazine

2. UV absorbers and light stabilizers

2.1. 2-(2'-Hydroxyphenyl)-benzotriazoles, for example, the 5'-methyl-, 3',5'-di-tert-butyl-,5'-tert-butyl-, 5'-(1,1,3,3-tetramethylbutyl)-, 5-chloro-3',5'-di-tert-butyl-, 5-chloro-3'-tert-butyl-5'-methyl-, 3'-sec-butyl-5'-tert-butyl-, 4'-octoxy, 3',5'-di-tert-amyl-, 3',5'-bis-(α,α-dimethylbenzyl), 3'-tert-butyl-5'-(2-(omega-hydroxy-octa-(ethyleneoxy)carbonyl-ethyl)-, 3'-dodecyl-5'-methyl-, and 3'-tert-butyl-5'-(2-octyloxycarbonyl)ethyl-, and dodecylated-5'-methyl derivatives.

2.2. 2-Hydroxy-benzophenones, for example, the 4-hydroxy-, 4-methoxy-, 4-octoxy, 4-decyloxy-, 4-dodecyloxy-, 4-benzyloxy, 4,2',4'-trihydroxy- and 2'-hydroxy-4,4'-dimethoxy derivatives.

2.3. Esters of optionally substituted benzoic acids for example, phenyl salicylate, 4-tert-butylphenyl salicylate, octylphenyl salicylate, dibenzoylresorcinol, bis-(4-tert-butylbenzoyl)-resorcinol, benzoylresorcinol, 3,5-di-tert-butyl-4-hydroxybenzoic acid 2,4-di-tert-butylphenyl ester and 3,5-di-tert-butyl-4-hydroxybenzoic acid hexadecyl ester.

2.4. Acrylates, for example, α-cyano-β,β-diphenylacrylic acid ethyl ester or isooctyl ester, α-carbomethoxy-cinnamic acid methyl ester, α-cyano-β-methyl-p-methoxy-cinnamic acid methyl ester or butyl ester, α-carbomethoxy-p-methoxy-cinnamic acid methyl ester, N-(β-carbomethoxy-β-cyanovinyl)-2-methyl-indoline.

2.5. Nickel compounds, for example, nickel complexes of 2,2'-thio-bis-[4-(1,1,3,3-tetramethylbutyl)-phenol], such as the 1:1 or 1:2 complex, optionally with additional ligands such as n-butylamine, triethanolamine or N-cyclohexyl-diethanolamine, nickel dibutyldithiocarbamate, nickel salts of 4-hydroxy-3,5-di-tert-butylbenzylphosphonic acid monoalkyl esters, such as of the methyl, ethyl or butyl ester, nickel complexes of ketoximes such as of 2-hydroxy-4-methyl-phenyl undecyl ketoxime, nickel complexes of 1-phenyl-4-lauroyl-5-hydroxy-pyrazole, optionally with additional ligands.

2.6. Sterically hindered amines, for example bis-(2,2,6,6-tetramethylpiperidyl) sebacate, bis-(1,2,2,6,6-pentamethylpiperidyl) sebacate, n-butyl-3,5-di-tert.butyl-4-hydroxybenzyl malonic acid bis-(1,2,2,6,6-pentanemethylpiperidyl)ester, condensation product of 1-hydroxyethyl-2,2,6,6-tetramethyl-4-hydroxypiperidine and succinic acid, condensation product of N,N'-(2,2,6,6-tetramethylpiperidyl)-hexamethylenediamine and 4-tert-octylamino-2,6-dichloro-s-triazine, tris-(2,2,6,6-tetramethylpiperidyl)-nitrilotriacetate, tetrakis-(2,2,6,6-tetramethyl-4-piperidyl)-1,2,3,4-butane-tetracarbonic acid, 1,1'(1,2-ethanedyl)-bis-(3,3,5,5-tetramethylpiperazinone).

2.7. Oxalic acid diamides, for example, 4,4'-di-octyloxy-oxanilide, 2,2'-di-octyloxy-5,5'-di-tert-butyl-oxanilide, 2,2'-di-dodecyloxy-5,5'-di-tert-butyl-oxanilide, 2-ethoxy-2'-ethyl-oxanilide, N,N'-bis (3-dimethylaminopropyl)-oxalamide, 2-ethoxy-5-tert-butyl-2'-ethyloxanilide and its mixture with 2-ethoxy-2'-ethyl-5,4'-di-tert-butyloxanilide and mixtures of ortho- and para-methoxy- as well as of o- and p-ethoxy-disubstituted oxanilides.

2.8. Hydroxyphenyl-s-triazines, for example 2,6-bis-(2,4-dimethylphenyl)-4-(2-hydroxy-4-octyloxyphenyl)-s-triazine; 2,6-bis-(2,4-dimethylphenyl)-4-(2,4-dihydroxyphenyl)-s-triazine; 2,4-bis(2,4-dihydroxyphenyl)-6-(4-chlorophenyl)-s-triazine; 2,4-bis[2-hydroxy-4-(2-hydroxyethoxy)phenyl]-6-(4-chlorophenyl)-s-triazine; 2,4-bis[2-hydroxy-4-(2-hydroxy-4-(2-hydroxyethoxy)phenyl]-6-(2,4-dimethylphenyl)-s-triazine; 2,4-bis[2-hydroxy-4-(2-hydroxyethoxy)-phenyl]-6-(4-bromophenyl)-s-triazine; 2,4-bis[2-hydroxy-4-(2-acetoxyethoxy)phenyl]-6-(4-chlorophenyl)-s-triazine, 2,4-bis(2,4-dihydroxyphenyl)-6-(2,4-dimethylphenyl)-s-triazine.

3. Metal deactivators, for example, N,N'-diphenyloxalic acid diamide, N-salicylal-N'-salicyloylhydrazine, N,N'-bis-salicyloylhydrazine, N,N'-bis-(3,5-di-tert-butyl-4-hydroxyphenylpropionyl)-hydrazine, 3-salicyloylamino 1,2,4-triazole, bis-benzylidene-oxalic acid dihydrazide.

4. Phosphites and phosphonites, for example, triphenyl phosphite, diphenylalkyl phosphites, phenyldiakyl phosphites, tri-(nonylphenyl) phosphite, trilauryl phosphite, trioctadecyl phosphite, di-stearyl-pentaerythritol diphosphite, tris-(2,4-di-tert-butylphenyl) phosphite, di-isodecylpentaethritol diphosphite, di-(2,4-di-tert-butylphenyl)pentaethritol diphosphite, tristearyl-sorbitol triphosphite, tetrakis-(2,4-di-tert-butylphenyl) 4,4'-diphenylylenediphosphonite.

5. Compounds which destroy peroxide, for example, esters of β-thiodipropionic acid, for example the lauryl, stearyl, myristyl or tridecyl esters, mercapto-benzimidazole or the zinc salt of 2-mercaptobenzimidazole, zinc dibutyl-dithiocarbamate, dioctadecyl disulfide, pentaerythritol tetrakis-(β-dodecylmercapto)-propionate.

6. Hydroxylamines, for example, N,N-dibenzylhydroxylamine, N,N-diethylhydroxylamine, N,N-dioctylhydroxylamine, N,N-dilaurylhydroxylamine, N,N-ditetradecylhydroxylamine, N,N-dihexadecylhydroxylamine, N,N-dioctadecylhydroxylamine, N-hexadecyl-N-octadecylhydroxylamine, N-heptadecyl-N-octadecylhydroxylamine, N,N-dialkylhydroxylamine derived from hydrogenated tallow amine.

7. Nitrones, for example, N-benzyl-alpha-phenyl nitrone, N-ethyl-alpha-methyl nitrone, N-octyl-alpha-heptyl nitrone, N-lauryl-alpha-undecyl nitrone, N-tetradecyl-alpha-tridecyl nitrone, N-hexadecyl-alpha-pentadecyl nitrone, N-octadecyl-alpha-heptadecylnitrone, N-hexadecyl-alpha-heptadecyl nitrone, N-octadecyl-alpha-pentadecyl nitrone, N-heptadecyl-alpha-heptadecyl nitrone, N-octadecyl-alpha-hexadecyl nitrone, nitrone derived from N,N-dialkylhydroxylamine derived from hydrogenated tallow amine.

8. Polyamide stabilizers, for example copper salts in combination with iodides and/or phosphorus compounds and salts of divalent manganese.

9. Basic co-stabilizers, for example, melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example Ca stearate, Zn stearate, Mg stearate, Na ricinoleate and K palmitate, antimony pyrocatecholate or zinc pyrocatecholate.

10. Nucleating agents, for example, 4-tert-butyl-benzoic acid, adipic acid diphenylacetic acid.

11. Fillers and reinforcing agents, for example, calcium carbonate, silicates, glass fibers, asbestos, talc, kaolin, mica, barium sulfate, metal oxides and hydroxides, carbon black, graphite.

12. Other additives, for example, plasticizers, lubricants, emulsifiers, pigments, optical brighteners, flameproofing agents, anti-static agents, blowing agents and thiosynergists such as dilauryl thiodipropionate or distearyl thiodipropionate. The lubricant composition may also contain other additives, such as rust inhibitors, viscosity regulators, pour point depressants, dispersing agents or detergents, said additives being widely known and used in lubricants.

The following examples will further illustrate the embodiments of the instant invention.

## Example 1

N-Octylthiomethyl-4,4'-di-tert.-octyl-diphenylamine

3.7 g (0.025 mole) octyl mercaptan, 9.8 g (0.025 mole) 4-di-tert.octyldiphenylamine and 2.5 g of a 37 % aqueous formaldehyde solution (0.03 mole) in 100 ml methanol are refluxed for 3 hours during which time an oily mixture is formed. Refluxing is continued for 3-4 hours. The mixture is then allowed to stand overnight at room temperature whereupon the reaction mixture is then again refluxed for 4 hours. The reaction progess is monitored by thin layer (TLC). After completing the reaction, the mixture is concentrated by evaporation and the product is isolated. One isolates 1.7g of the title compound of the formula

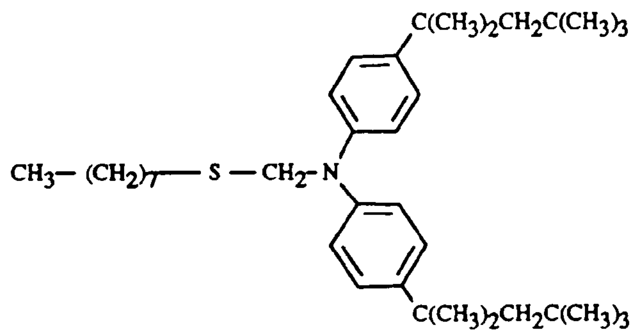

| Microanalysis (C/H/N/S in %): | | | | |
|---|---|---|---|---|
| Calc'd for $C_{37}H_{61}NS$ : | 80.51; | 11.14; | 2.54; | 5.8 |
| Found: | 80.6 | 11.3; | 2.7; | 5.5 |

The following compounds are prepared according to Example 1:

**TABLE 1**

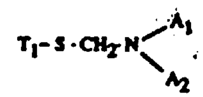 (I)

| Compound No. | $T_1$ | $A_1$ & $A_2$ | Microanalysis C/H/N/S/ (%) |
|---|---|---|---|
| 2 | $CH_3(CH_2)_7$- | Phenyl | calc: 77.01/8.93/4.28/--<br>found: 77.2/9.1/4.1/-- |
| 3 | $CH_3(CH_2)_7$- | mixture of t-butylphenyl and t-octylphenyl | calc: 79.77/10.67/2.91/6.65<br>found: 79.5/10.3/2.9/6.6<br>(for $C_{32}H_{51}NS$, ave. comp.) |
| 4 | Phenyl | Phenyl | calc: 78.3, 5.88, 4.81, 11.0<br>found: 78.17, 6.08, 4.76, 11.06<br>mp. 72-76 |
| 5 | $CH_3(CH_2)_{11}$- | Phenyl | oil |
| 6 | $-CH_2CH_2OH$ | mixture of t-butylphenyl and t-octylphenyl | calc: 74.34/8.95/3.77/8.63<br>found: 74.1/9.37/3.50/8.45 |
| 7 | tert.dodecyl | Phenyl | Oil |
| 8 | tert.nonyl | Phenyl | Oil |

**TABLE 1( Continued)**

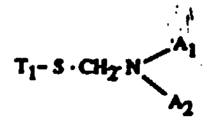 (I)

| Compound No. | $T_1$ | $A_1$ & $A_2$ | Microanalysis C/H/N/S/ (%) |
|---|---|---|---|
| 9 | $CH_3(CH_2)_7$- | $A_1$ & A2 together: 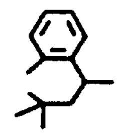 | calc: 75.61/0.58/4.20/9.61<br>found: 75.44/10.67/4.29/9.29 |
| 10 | $CH_3(CH_2)_7$- | $A_1$ : Phenyl $A_2$ : $-CH_2SC_8H_{17}$ | calc: 70.35/10.58/3.42/15.65<br>found: 70.33/10.43/3.65/15.44 |
| 11 | $CH_3(CH_2)_{11}$- | $A_1$ : 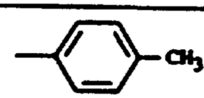 $A_2$ : $-CH_2SC_{12}H_{25}$ | calc: 73.95/11.47/2.63/11.96<br>found: 73.91/11.45/2.73/11.46 |
| 12 | $CH_3(CH_2)_7$- | $A_1$ : 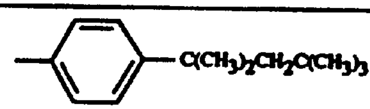<br>$A_2$ : Naphthyl | calc: 80.92/9.67/2.86/6.55<br>found: 81.14/9.64/2.84/6.18 |
| 13 | tert.nonyl | $A_1$: 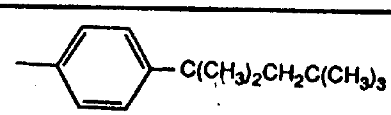<br>$A_2$: Naphthyl. | — |

## TABLE 2

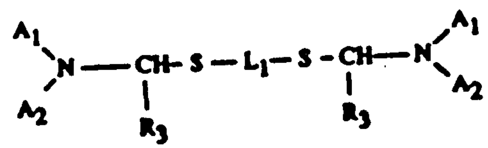

(II)

| Compound No | $A_1$ & $A_2$ | $R_3$ | $L_1$ | Physical data Microanalysis (C/H/N/S , % ) |
|---|---|---|---|---|
| 14 | $(CH_3)_3CCH_2(CH_3)_2C$- 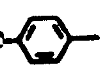 | H | $-(CH_2)_6-$ | mp 93-95°C<br>Calc'd. for $C_{37}H_{61}NS$: 79.93/10.48/2.91/6.67<br>Found: 80.2/10.6/2.9/6.6 |
| 15 | Phenyl | H | $-(CH_2)_6-$ | mp 44-47°C<br>Calc: 74.95/7.08/5.46/12.50<br>Found: 74.7/7.5/5.5/12.8 |

TABLE 3

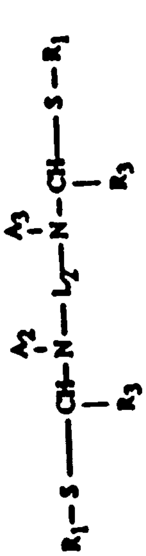

(III)

| Compound No. | $R_1$ | $R_3$ | $A_2$ & $A_3$ | $L_2$ | Physical data Microanalysis (C/H/N/S [%]) |
|---|---|---|---|---|---|
| 16 | $CH_3(CH_2)_7$- | H | $CH_3(CH_2)_7SCH_2$- | 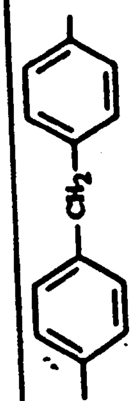 | oil<br>calc: 70.78/10.43/3.37/15.42<br>found: 70.5/10.6/3.3/15.4 |
| 17 | $CH_3(CH_2)_7$- | H | Phenyl | 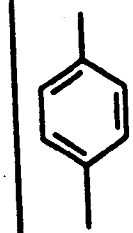 | oil<br>calc: -/-/-/11.12<br>found: -/-/-/10.7 |
| 18 | $CH_3(CH_2)_7$- | H | $A_2$: Phenyl<br>$A_3$: $CH_3(CH_2)_7SCH_2$- | 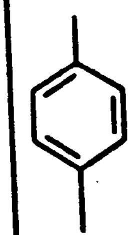 | oil<br>calc: 71.07/10.09/4.25/14.59<br>found: 71.05/10.07/4.5/14.31 |
| 19 | tert.dodecyl | H | Phenyl | 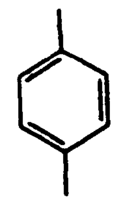 | — |

Example 20

Octanethiol (14.6 g., 0. 10 mol), 4,4'-methylenedianiline (9.9 g., 0.05 mol) and 37 % aqueous formaldehyde solution (22.7 g, 0.28 mol) in 150 ml methanol are refluxed for 7 hours during which time an oily mixture is

formed. The the mixture is allowed to stand overnight at room temperature. After completing the reaction, the mixture is concentrated by evaporation and the product is isolated.

Example 21

The following compounds can be prepared utilizing similar synthetic procedures:

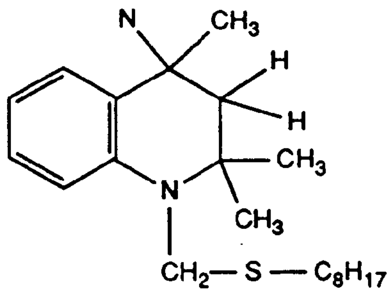
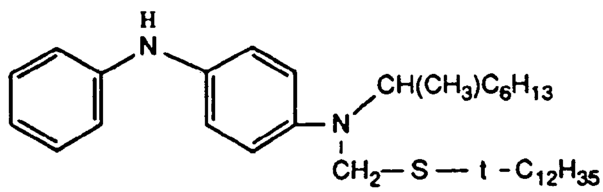

Example 22

A 150 N paraffinic mineral oil (50 grams) containing 0.25 % by weight of a test compound and 5 mL of water is added to a galss container having a copper coil which is housed inside a bomb fitted with a pressure gauge as described in ASTM D 2272. The bomb is charged with oxygen to a pressure of 90 psi (620 kPa), placed in a constant temperature oil bath at 150 °C and rotated axially at 100 rpm at a 30° angle from the horizontal. The time required for the test sample to react with a given volume of oxygen is measured, completion of reaction being indicated by a specific drop in pressure (25 psi, 172 kPa) from the plateau pressure originally observed. This time is reported as minutes to failure

| Additive | Additive Conc. (% by weight) | Minutes to Failure |
|---|---|---|
| None (Blank Oil) | - | 31 |
| Example 1 | 0.25 | 127 |
| Example 4 | 0.25 | 68 |
| Example 16 | 0.25 | 70 |

Example 23

Engine Oil Thin Film Oxygen Uptake Test

The antioxidant effectiveness of the instant stabilizers in engine oils is evaluated by ASTM test method, D4742. A 1.5 gram test sample of a 10W-30 engine oil, formulated to meet SD/CC quality level, containing 0.5% by weight of the test compound is placed in the test apparatus. The test is then completed according to the standard method procedure and the oxidation induction time, in minutes, is determined (reported in the table below). A longer induction time indicates greater oxidation stability.

| Additive | Additive Conc. (% by weight) | Minutes to Failure |
|---|---|---|
| None (Blank Oil) | - | 110 |
| Example 3 | 0.5 | 124 |
| Example 4 | 0.5 | 203 |
| Example 17 | 0.5 | 213 |

Example 24

This example illustrates the process stabilizing effectiveness of the instant stabilizers in elastomers.

The stabilizer is evaluated in polybutadiene at 0.25 % by weight. After incorporation of the stabilizer into the elastomer by way of a cyclohexane solution, the stabilized elastomer is isolated by steam coagulation. The crumb is then washed with water and dried under vacuum at 40 °C. High temperature aging is conducted in a Brabender Plasticorder at 160 °C and 60 RPM for 30 min. Stabilization is measured by the length of time it takes for an increase in torque to take place (= cross-linking). A highly stabilized elastomer has a long induction time.

| Additive | Induction Time |
|---|---|
| None (Blank Oil) | 13 |
| Example 5 | >30 |

## Claims

1. A composition stabilized against degradation which comprises
(a) an organic material subject to oxidative, thermal or light induced degradation and
(b) as stabilizer, an effective stabilizing amount of a compound of formulae A-H

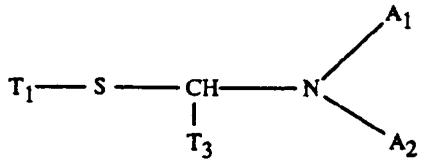

(A)

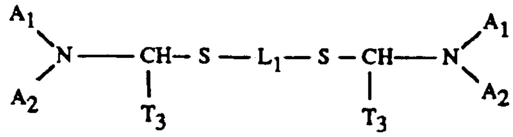

(B)

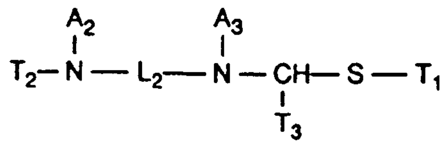

(C)

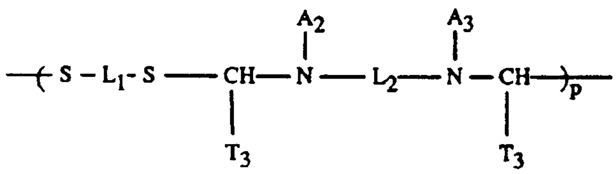

(D)

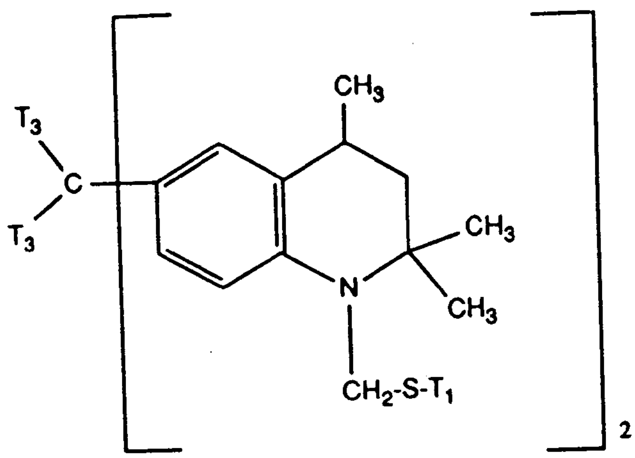

(E)

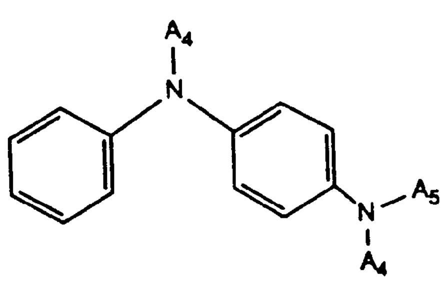

(F)

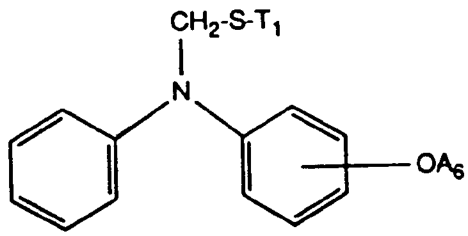

(G)

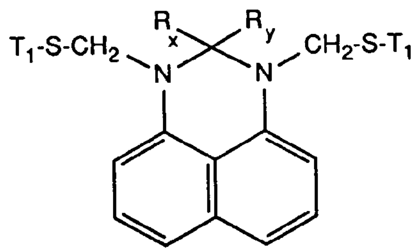

(H)

wherein

$T_1$ is $C_6$-$C_{12}$aryl, $C_1$-$C_{30}$alkyl, $C_5$-$C_{12}$cycloalkyl or $-CH_2CH_2OH$;

$T_2$ is

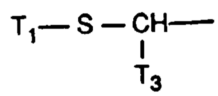

or H when $A_2$ is phenyl;

$T_3$ is H, $C_1$-$C_{14}$alkyl, phenyl or phenyl-$C_1$-$C_4$alkyl;

$A_1$, $A_2$ and $A_3$ independently of each other are H, $C_1$-$C_{18}$alkyl,

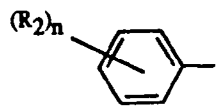

,

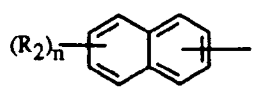

,

or $-CH(T_3)$-S-$T_1$; or

$A_1$ and $A_2$ together with the nitrogen atom to which they are attached are

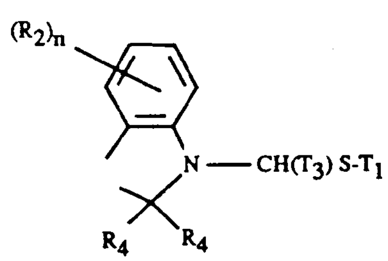

,

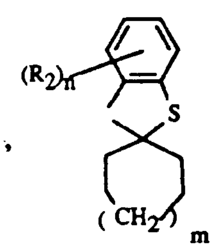

,

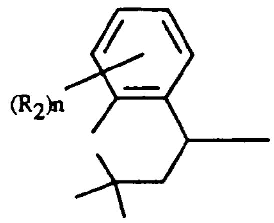 , 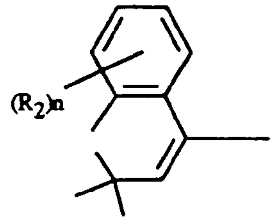 , or 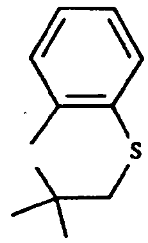 ;

$R_2$ is H, $C_1$-$C_{30}$alkyl or -C($R_6$)($R_7$)phenyl;
$R_4$ is $C_1$-$C_{16}$alkyl or two $R_4$ groups together are pentamethylene;
$R_6$ and $R_7$ are independently H or phenyl;
$R_x$ and $R_y$ are independently H, $C_1$-$C_{18}$alkyl, $C_5$-$C_{12}$cycloalkyl or phenyl;
$A_4$ independently is H or -$CH_2$-S-$T_1$, at least one of $A_4$ being -$CH_2$-S-$T_1$;
$A_5$ is H, $C_1$-$C_{16}$alkyl, $C_6$-$C_{12}$aryl or -$CH_2$-S-$T_1$;
$A_6$ is H or $C_1$-$C_{25}$alkyl;
m is 1 or 2;
n is 1, 2, 3 or 4;
p is 1 to 20;
$L_1$ is a direct bond, linear or branched $C_2$-$C_{12}$alkylene, $C_6$-$C_{12}$arylene, or

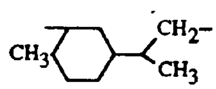 ;

and
$L_2$ is $C_2$-$C_{12}$alkylene, $C_6$-$C_{12}$arylene, xylylene, $C_4$-$C_{12}$alkylene which is interrupted by -N(CH($T_3$)-S-$T_1$) or $L_2$ is

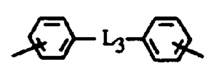

wherein $L_3$ is a direct bond, $C_1$-$C_6$alkylene, S, -NH- or O.

2. A composition according to claim 1, wherein $T_1$ is $C_8$-$C_{12}$alkyl.

3. A composition according to claim 1, wherein $T_1$ is -$CH_2CH_2OH$.

4. A compositon according to claim 1, wherein $T_2$ is

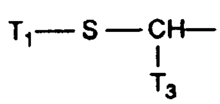 .

5. A compositon according to claim 1, wherein $T_2$ is H and A is phenyl.

6. A composition according to claim 1, wherein $T_3$ is H.

7. A composition according to claim 2, wherein $A_1$ and $A_2$ are

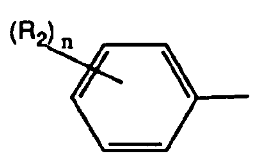 or 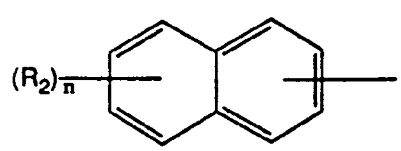

in which $R_2$ is independently H or $C_1$ -$C_{30}$alkyl which can be linear or branched and in which n is 1, 2, 3 or 4.

**8.** A composition according to claim 7, wherein $R_2$ is tert.-butyl or tert.-octyl.

**9.** A composition according to claim 7, wherein $A_1$ and $A_2$ are the same

**10.** A composition according to claim 1, wherein $L_2$ is phenylene.

**11.** A composition according to claim 1, wherein said compound corresponds to formula (E) and $T_1$ is octyl and $T_3$ is hydrogen.

**12.** A composition according to claim 1, wherein the organic material to be stabilized is a lubricant.

**13.** A composition according to claim 1, wherein the organic material to be stabilized is an elastomer.

**14.** A compound corresponding to formulae I-VIII:

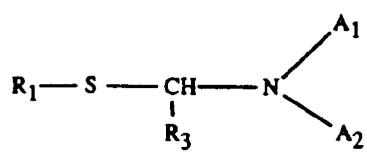

(I)

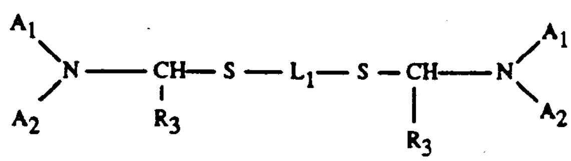

(II)

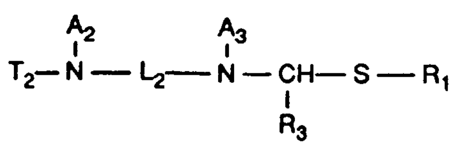

(III)

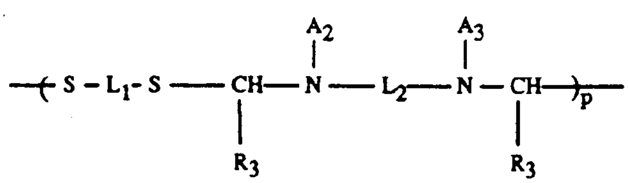

(IV)

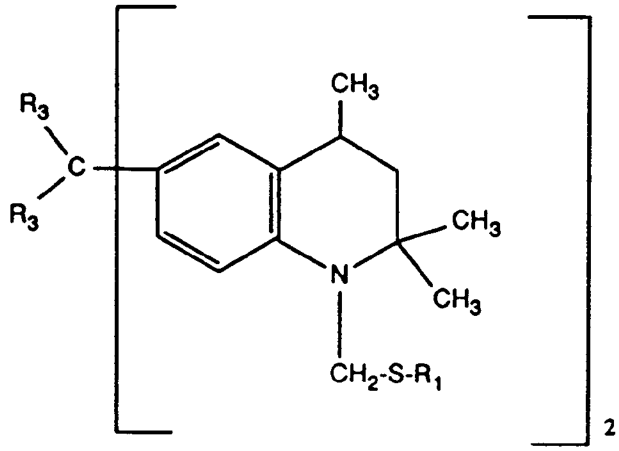

(V)

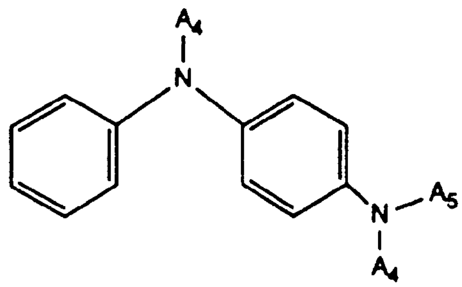

(VI)

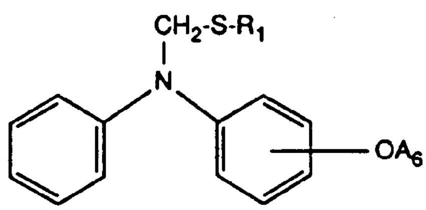

(VII)

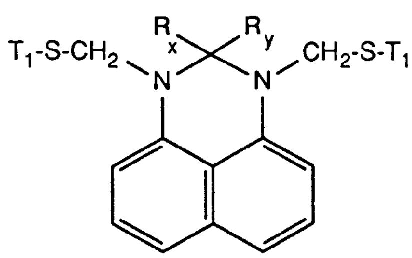

(VIII)

wherein
$T_1$, $R_x$ and $R_y$ are as defined in claim 1,
$R_1$ is $C_1$-$C_{30}$alkyl, $C_5$-$C_{12}$-cycloalkyl, phenyl or $-CH_2CH_2OH$;
$T_2$ is

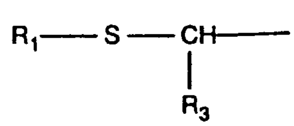

or H when $A_2$ is phenyl;
$R_3$ is H, $C_1$-$C_{14}$alkyl, phenyl or phenyl-$C_1$-$C_4$alkyl;
$R_4$ is $C_1$-$C_{16}$alkyl or two $R_4$ groups together are pentamethylene;
$A_1$, $A_2$ and $A_3$, independently of each other, are H, $C_1$-$C_{18}$alkyl,

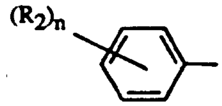
,

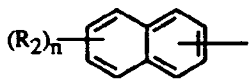
,

or -CH($R_3$)-S-$R_5$ wherein $R_2$ is H, $C_1$-$C_{30}$alkyl or -C($R_6$)($R_7$)phenyl, and $R_5$ is $C_6$-$C_{12}$aryl, $C_1$-$C_{30}$alkyl, $C_5$-$C_{12}$cycloalkyl or -$CH_2CH_2OH$; or
$A_1$ and $A_2$ together with the nitrogen atom to which they are attached are

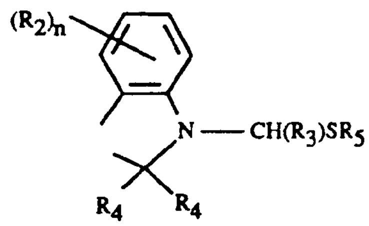
or
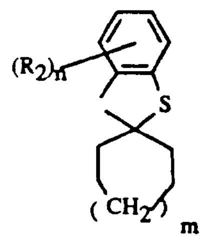
,

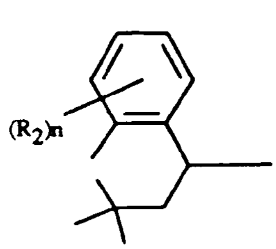
,
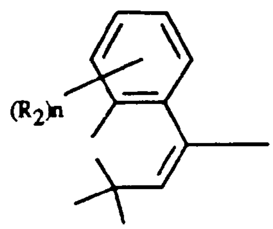
, or
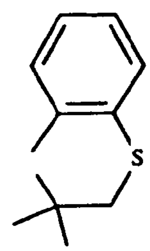
;

wherein
$A_4$ independently is H or -$CH_2$-S-$R_1$, at least one of $A_4$ being -$CH_2$-S-$R_1$;
$A_5$ is H, $C_1$-$C_{16}$alkyl, $C_6$-$C_{12}$aryl or -$CH_2$-S-$R_1$;
$A_6$ is H or $C_1$-$C_{25}$alkyl;
m is 0, 1 or 2;
n is 1, 2, 3 or 4;
p is 1 to 20;
$L_1$ is a direct bond, linear or branched $C_2$-$C_{12}$alkylene, $C_6$-$C_{12}$arylene, or

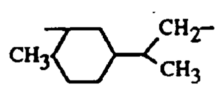

and
$L_2$ is $C_2$-$C_{12}$alkylene, $C_6$-$C_{12}$arylene, xylylene, $C_4$-$C_{12}$alkylene which is interrupted by -N(CH($R_3$)-S-$R_5$) wherein $R_5$ is as defined above or $L_2$ is

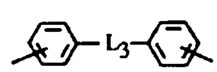

wherein $L_3$ is a direct bond, $C_1$-$C_6$alkylene, S, -NH- or O.

15. A compound according to claim 14, wherein $L_2$ is phenylene or

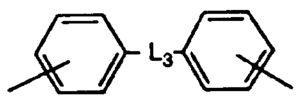

in which $L_3$ is $C_1$-$C_6$alkylene, S, -NH-, O or a direct bond.

16. A method of stabilizing an organic material subject to oxidative, thermal or light induced degradation, which comprises incorporating into said material at least one compound of formula (A) to (H) as defined in claim 1.

**Patentansprüche**

1. Masse, stabilisiert gegen Abbau, umfassend (a) ein organisches oxidativem, thermischem oder lichtinduziertem Abbau unterliegendes Material und (b) als Stabilisator eine wirksam stabilisierende Menge einer Verbindung der Formeln A-H

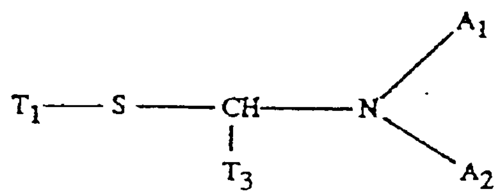

(A)

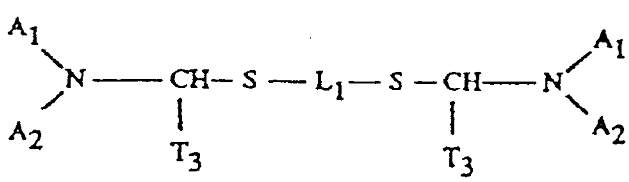

(B)

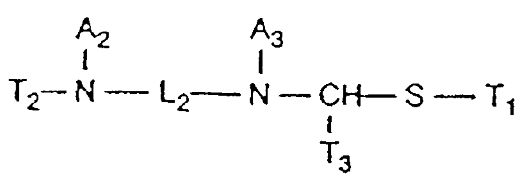

(C)

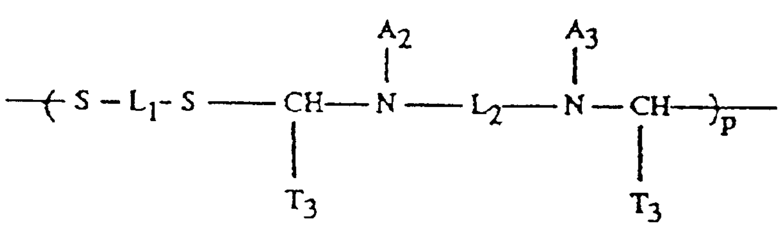

(D)

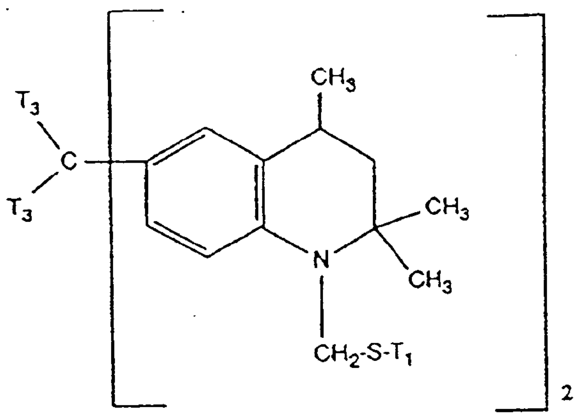

(E)

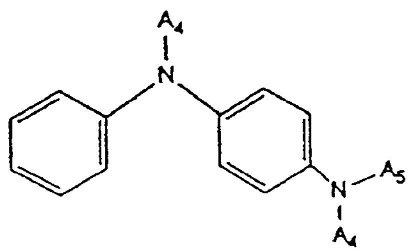

(F)

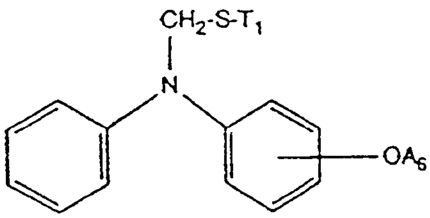

(G)

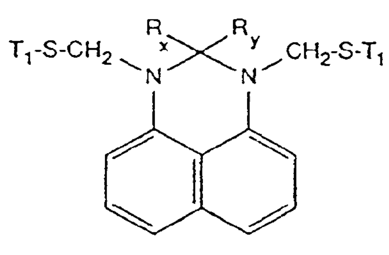

(H)

worin

$T_1$ $C_6$-$C_{12}$-Aryl, $C_1$-$C_{30}$-Alkyl, $C_5$-$C_{12}$-Cycloalkyl oder $-CH_2CH_2OH$ bedeutet;

$T_2$

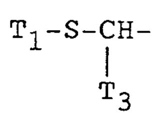

oder H bedeutet, wenn $A_2$ Phenyl darstellt;

$T_3$ H, $C_1$-$C_{14}$-Alkyl, Phenyl oder Phenyl-$C_1$-$C_4$-Alkyl bedeutet;

$A_1$, $A_2$ und $A_3$ unabhängig voneinander H, $C_1$-$C_{18}$-Alkyl,

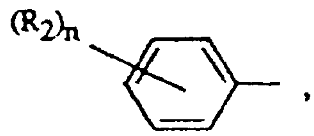

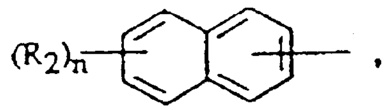

oder $-CH(T_3)-S-T_1$ bedeuten; oder

$A_1$ und $A_2$ zusammen mit dem Stickstoffatom, an das sie gebunden sind

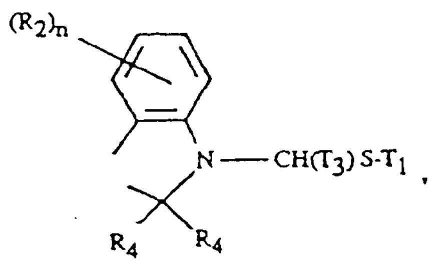

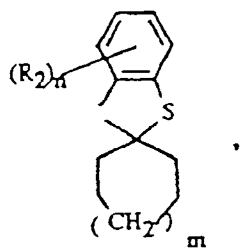

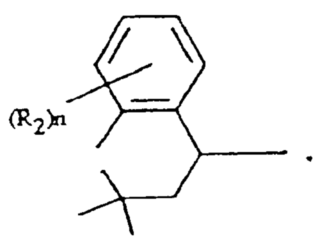

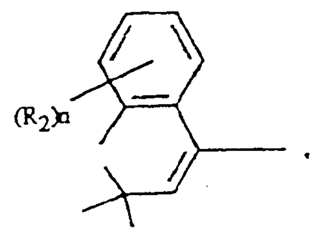

oder

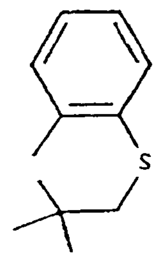

bedeuten;

$R_2$ H, $C_1$-$C_{30}$-Alkyl oder $-C(R_6)(R_7)$-Phenyl bedeutet;

$R_4$ $C_1$-$C_{16}$-Alkyl bedeutet oder zwei $R_4$-Gruppen zusammen Pentamethylen darstellen;

$R_6$ und $R_7$ unabhängig voneinander H oder Phenyl bedeuten;

$R_x$ und $R_y$ unabhängig voneinander H, $C_1$-$C_{18}$-Alkyl, $C_5$-$C_{12}$-Cycloalkyl oder Phenyl darstellen;

$A_4$ unabhängig voneinander H oder $-CH_2-S-T_1$ bedeutet, wobei mindestens ein Rest $A_4$ $-CH_2-S-T_1$ darstellt;

$A_5$ H, $C_1$-$C_{16}$-Alkyl, $C_6$-$C_{12}$-Aryl oder $-CH_2-S-T_1$ darstellt;

$A_6$ H oder $C_1$-$C_{25}$-Alkyl darstellt;

m 1 oder 2 ist;

n 1, 2, 3 oder 4 ist;

p 1 bis 20 bedeutet;

$L_1$ eine direkte Bindung, linear oder verzweigt, $C_2$-$C_{12}$-Alkylen, $C_6$-$C_{12}$-Arylen oder

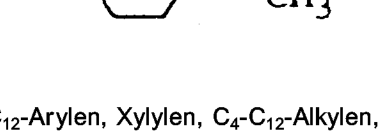

bedeutet und

$L_2$ $C_2$-$C_{12}$-Alkylen, $C_6$-$C_{12}$-Arylen, Xylylen, $C_4$-$C_{12}$-Alkylen, unterbrochen durch $-N(CH(T_3)-S-T_1)$, bedeutet oder $L_2$

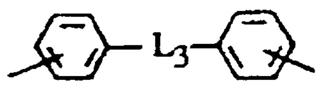 ,

bedeutet, worin $L_3$ eine direkte Bindung, $C_1$-$C_6$-Alkylen, S, -NH- oder O darstellt.

2. Masse nach Anspruch 1, wobei $T_1$ $C_8$-$C_{12}$-Alkyl darstellt.

3. Masse nach Anspruch 1, wobei $T_1$ $-CH_2CH_2OH$ darstellt.

4. Masse nach Anspruch 1, wobei $T_2$

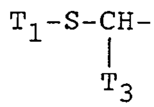

darstellt.

5. Masse nach Anspruch 1, wobei $T_2$ H bedeutet und A Phenyl ist.

6. Masse nach Anspruch 1, wobei $T_3$ H bedeutet.

7. Masse nach Anspruch 2, wobei $A_1$ und $A_2$

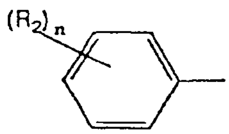 oder 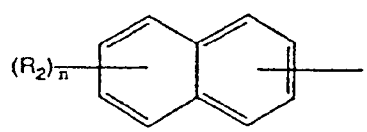

darstellen, wobei $R_2$ unabhängig voneinander H oder einen $C_1$-$C_{30}$-Alkylrest darstellt, der linear oder verzweigt sein kann, und worin n 1, 2, 3 oder 4 bedeutet.

8. Masse nach Anspruch 7, wobei $R_2$ tert.-Butyl oder tert.-Octyl darstellt.

9. Masse nach Anspruch 7, wobei $A_1$ und $A_2$ gleich sind.

10. Masse nach Anspruch 1, wobei $L_2$ Phenylen bedeutet.

11. Masse nach Anspruch 1, wobei die Verbindung Formel (E) entspricht und $T_1$ Octyl darstellt und $T_3$ Wasserstoff ist.

12. Masse nach Anspruch 1, wobei das organische zu stabilisierende Material ein Schmiermittel ist.

13. Masse nach Anspruch 1, wobei das organische zu stabilisierende Material ein Elastomer ist.

14. Verbindung gemäß Formeln I-VIII:

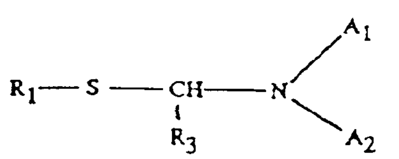 (I)

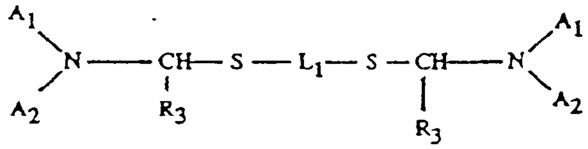

(II)

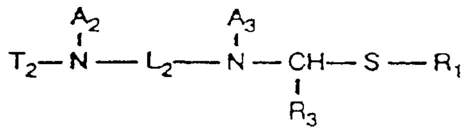

(III)

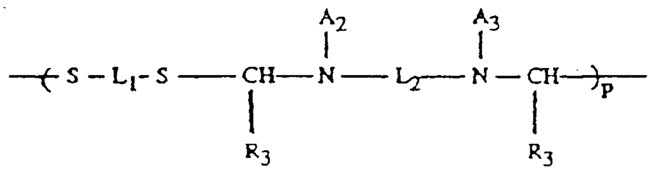

(IV)

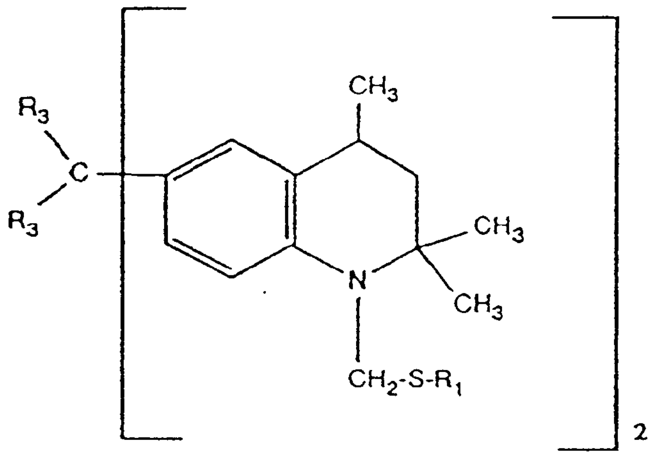

(V)

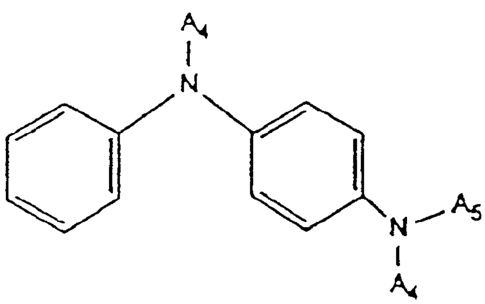

(VI)

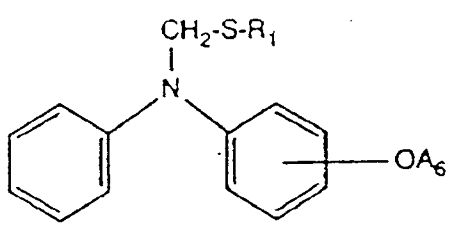

(VII)

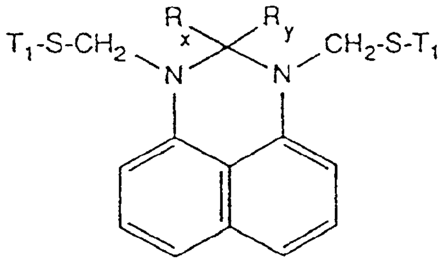

(VIII)

worin

$T_1$, $R_x$ und $R_y$ wie in Anspruch 1 definiert sind,

$R_1$ $C_1$-$C_{30}$-Alkyl, $C_5$-$C_{12}$-Cycloalkyl, Phenyl oder $-CH_2CH_2OH$ bedeutet;

$T_2$

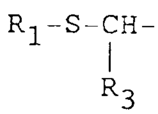

oder H bedeutet, wenn $A_2$ Phenyl darstellt;

$R_3$ H, $C_1$-$C_{14}$-Alkyl, Phenyl oder Phenyl-$C_1$-$C_4$-Alkyl bedeutet;

$R_4$ $C_1$-$C_{16}$-Alkyl bedeutet oder zwei $R_4$-Gruppen zusammen Pentamethylen darstellen;

$A_1$, $A_2$ und $A_3$ unabhängig voneinander H, $C_1$-$C_{18}$-Alkyl,

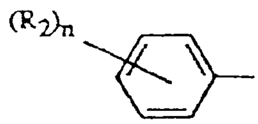
,

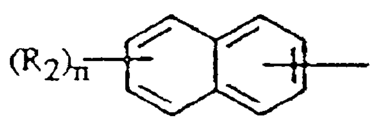
,

oder $-CH(R_3)-S-R_5$ bedeuten, wobei $R_2$ H, $C_1$-$C_{30}$-Alkyl oder $-C(R_6)(R_7)$-Phenyl darstellt und $R_5$ $C_6$-$C_{12}$-Aryl, $C_1$-$C_{30}$-Alkyl, $C_5$-$C_{12}$-Cycloalkyl oder $-CH_2CH_2OH$ bedeutet;

oder $A_1$ und $A_2$ zusammen mit dem Stickstoffatom, an das sie gebunden sind

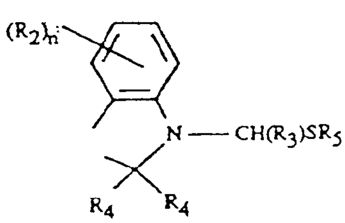
oder
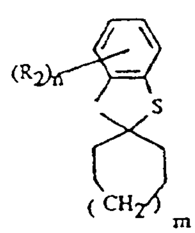
,

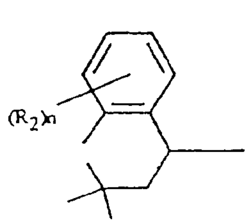
,
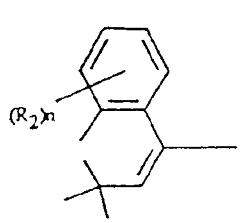
oder
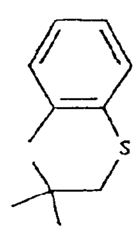

bedeuten;
worin
$A_4$ unabhängig voneinander H oder $CH_2$-S-$R_1$ bedeutet, wobei mindestens ein Rest $A_4$ $CH_2$-S-$R_1$ darstellt;
$A_5$ H, $C_1$-$C_{16}$-Alkyl, $C_6$-$C_{12}$-Aryl oder -$CH_2$-S-$R_1$ darstellt;
$A_6$ H oder $C_1$-$C_{25}$-Alkyl darstellt;
m 0, 1 oder 2 ist;
n 1, 2, 3 oder 4 ist;
p 1 bis 20 bedeutet;
$L_1$ eine direkte Bindung, linear oder verzweigt, $C_2$-$C_{12}$-Alkylen, $C_6$-$C_{12}$-Arylen oder

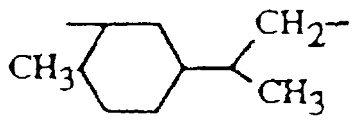

bedeutet und
$L_2$ $C_2$-$C_{12}$-Alkylen, $C_6$-$C_{12}$-Arylen, Xylylen, $C_4$-$C_{12}$-Alkylen, unterbrochen durch -N(CH($R_3$)-S-$R_5$) bedeutet, worin $R_5$ wie vorstehend definiert ist oder $L_2$

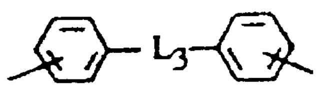

bedeutet, worin $L_3$ eine direkte Bindung, $C_1$-$C_6$-Alkylen, S, -NH- oder O darstellt.

**15.** Verbindung nach Anspruch 14, wobei $L_2$ Phenylen bedeutet oder

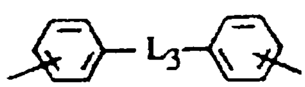

wobei $L_3$ $C_1$-$C_6$-Alkylen, S, -NH-, O oder eine direkte Bindung bedeutet.

**16.** Verfahren zum Stabilisieren eines organischen oxidativem, thermischem oder lichtinduziertem Abbau unterliegenden Materials, umfassend Einmischen mindestens einer Verbindung der Formel (A) bis (H) gemäß Anspruch 1 in das Material.

## Revendications

**1.** Composition stabilisée contre une dégradation qui comprend
(a) un substrat organique sujet à une dégradation provoquée par la lumière, la chaleur ou l'oxydation, et
(b) comme stabilisant, une quantité stabilisante efficace d'un composé des formules A à H :

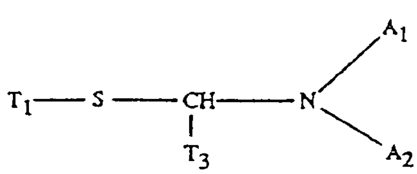

(A)

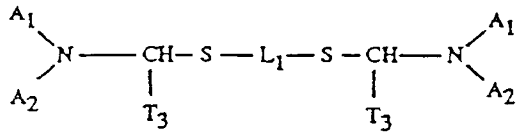

(B)

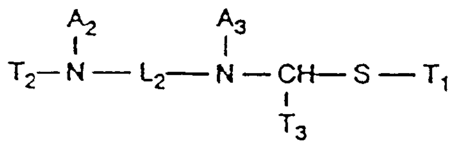

(C)

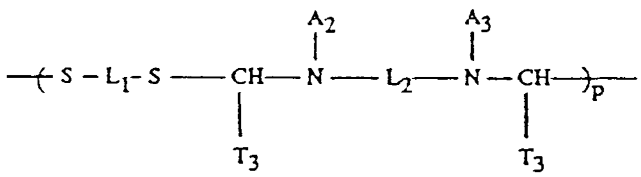

(D)

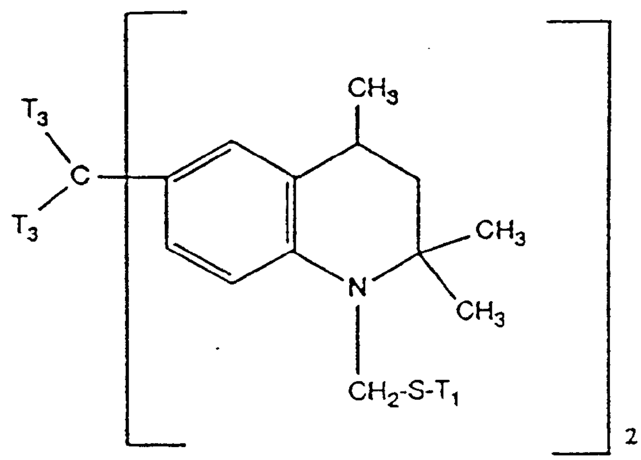

(E)

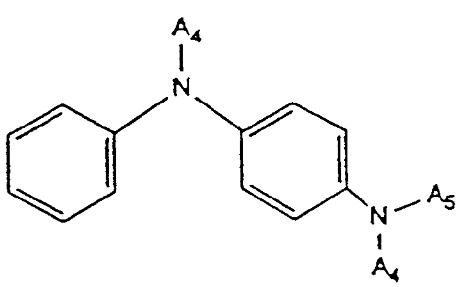

(F)

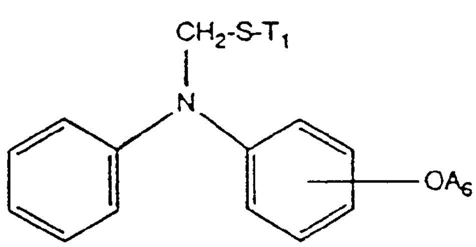

(G)

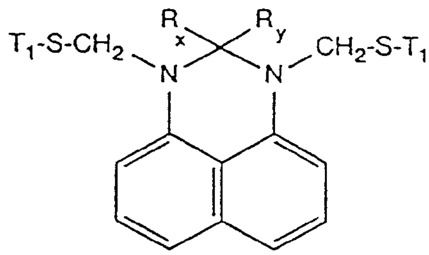

(H)

dans lesquelles

$T_1$ est un groupe aryle en $C_6$-$C_{12}$, alkyle en $C_1$-$C_{30}$, cycloalkyle en $C_5$-$C_{12}$ ou $-CH_2CH_2OH$ ;

$T_2$ est

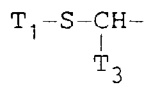

ou H lorsque $A_2$ est un groupe phényle ;

$T_3$ est H, un groupe alkyle en $C_1$-$C_{14}$, phényle ou phényl(alkyle en $C_1$-$C_4$) ;

$A_1$, $A_2$ et $A_3$ sont, chacun indépendamment des autres, H, un groupe alkyle en $C_1$-$C_{18}$,

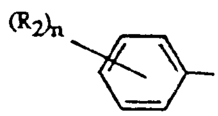 , 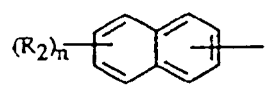

ou $-CH(T_3)$-S-$T_1$ ; ou bien

$A_1$ et $A_2$, avec l'atome d'azote auquel ils sont attachés, forment

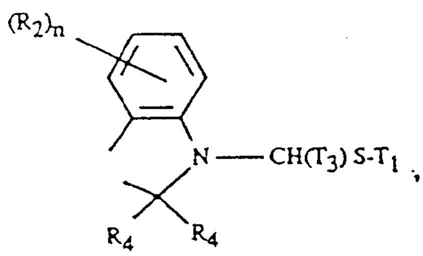 , 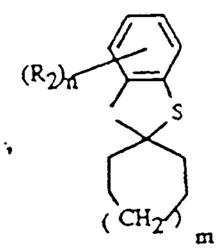 ,

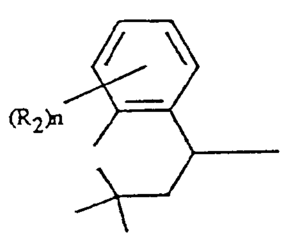 , 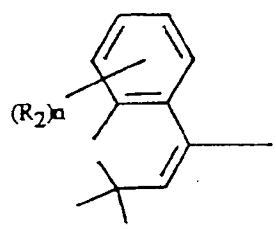 , ou 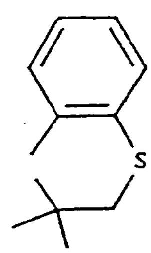 ;

$R_2$ est H, un groupe alkyle en $C_1$-$C_{30}$ ou $-C(R_6)(R_7)$-phényle ;

$R_4$ est un groupe alkyle en $C_1$-$C_{16}$ ou bien deux groupes $R_4$ forment ensemble un groupe pentaméthylène ;

$R_6$ et $R_7$ sont chacun indépendamment H ou un groupe phényle ;

$R_x$ et $R_y$ sont chacun indépendamment H, un groupe alkyle en $C_1$-$C_{18}$, cycloalkyle en $C_5$-$C_{12}$ ou phényle ;

$A_4$ est indépendamment H ou $-CH_2$-S-$T_1$, au moins l'un des $A_4$ étant $-CH_2$-S-$T_1$ ;

$A_5$ est H, un groupe alkyle en $C_1$-$C_{16}$, aryle en $C_6$-$C_{12}$ ou $-CH_2$-S-$T_1$ ;

$A_6$ est H ou un groupe alkyle en $C_1$-$C_{25}$ ;

m est 1 ou 2 ;
n est 1, 2, 3 ou 4 ;
p est 1 à 20 ;
$L_1$ est une liaison directe, un groupe alkylène en $C_2$-$C_{12}$ linéaire ou ramifié, arylène en $C_6$-$C_{12}$ ou

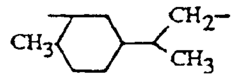 ;

et

$L_2$ est un groupe alkylène en $C_2$-$C_{12}$, arylène en $C_6$-$C_{12}$, xylylène, alkylène en $C_4$-$C_{12}$ qui est interrompu par -N(CH($T_3$)-S-$T_1$), ou bien $L_2$ est

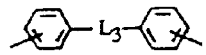

où $L_3$ est une liaison directe, un groupe alkylène en $C_1$-$C_6$, S, -NH- ou O.

**2.** Composition selon la revendication 1, dans laquelle $T_1$ est un groupe alkyle en $C_8$-$C_{12}$.

**3.** Composition selon la revendication 1, dans laquelle $T_1$ est -$CH_2CH_2OH$.

**4.** Composition selon la revendication 1, dans laquelle $T_2$ est

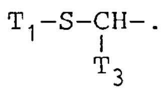

**5.** Composition selon la revendication 1, dans laquelle $T_2$ est H et A est un groupe phényle.

**6.** Composition selon la revendication 1, dans laquelle $T_3$ est H.

**7.** Composition selon la revendication 2, dans laquelle $A_1$ et $A_2$ sont

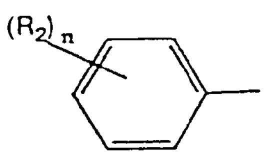 ou 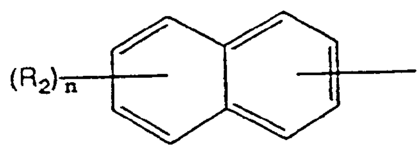

où $R_2$ est indépendamment H ou un groupe alkyle en $C_1$-$C_{30}$ qui peut être linéaire ou ramifié et où n est 1, 2, 3 ou 4.

**8.** Composition selon la revendication 7, dans laquelle $R_2$ est un groupe *tert*-butyle ou *tert*-octyle.

**9.** Composition selon la revendication 7, dans laquelle $A_1$ et $A_2$ sont identiques.

**10.** Composition selon la revendication 1, dans laquelle $L_2$ est un groupe phénylène.

**11.** Composition selon la revendication 1, dans laquelle ledit composé répond à la formule (E) et $T_1$ est un groupe octyle et $T_3$ est l'hydrogène.

**12.** Composition selon la revendication 1, dans laquelle la matière organique à stabiliser est un lubrifiant.

**13.** Composition selon la revendication 1, dans laquelle la matière organique à stabiliser est un élastomère.

**14.** Composé répondant aux formules I à VIII :

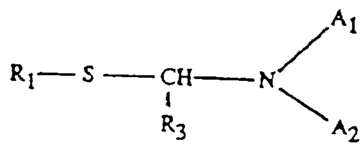

(I)

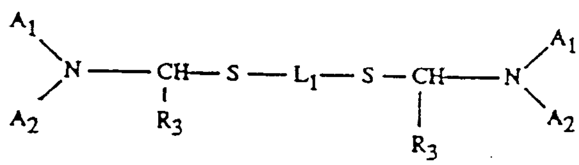

(II)

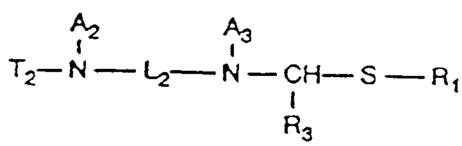

(III)

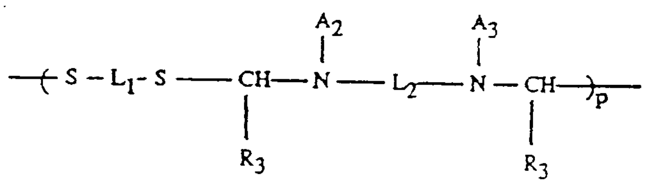

(IV)

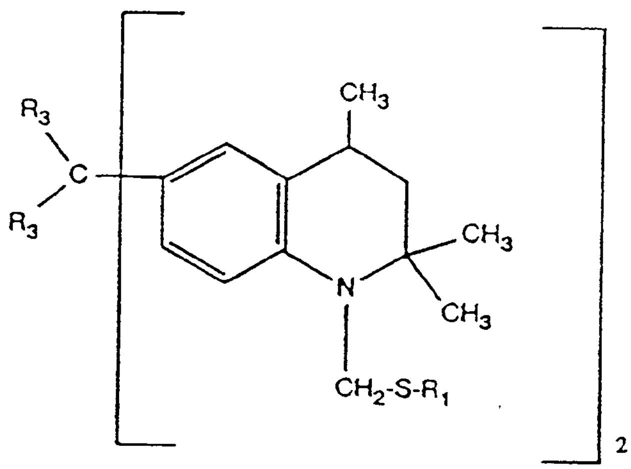

(V)

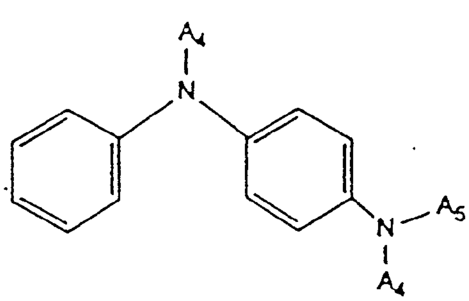

(VI)

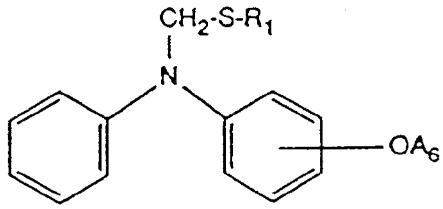

(VII)

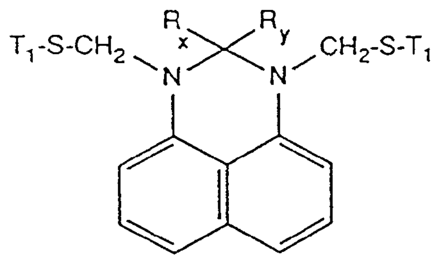

(VIII)

dans lesquelles

$T_1$, $R_x$ et $R_y$ sont tels que définis dans la revendication 1,

$R_1$ est un groupe alkyle en $C_1$-$C_{30}$, cycloalkyle en $C_5$-$C_{12}$, phényle ou $-CH_2CH_2OH$ ;

$T_2$ est

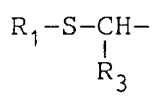

ou H lorsque $A_2$ est un groupe phényle ;

$R_3$ est H, un groupe alkyle en $C_1$-$C_{14}$, phényle ou phényl(alkyle en $C_1$-$C_4$) ;

$R_4$ est un groupe alkyle en $C_1$-$C_{16}$, ou bien deux groupes $R_4$ forment ensemble un groupe pentaméthylène ;

$A_1$, $A_2$ et $A_3$ sont, chacun indépendamment des autres, H, un groupe alkyle en $C_1$-$C_{18}$,

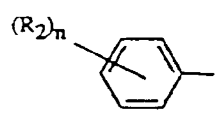 , 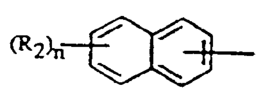

ou $-CH(R_3)$-S-$R_5$ où $R_2$ est H, un groupe alkyle en $C_1$-$C_{30}$ ou $-C(R_5)(R_7)$phényle, et $R_5$ est un groupe aryle en $C_6$-$C_{12}$, alkyle en $C_1$-$C_{30}$, cycloalkyle en $C_5$-$C_{12}$ ou $-CH_2CH_2OH$ ; ou bien

$A_1$ et $A_2$, avec l'atome d'azote auquel ils sont attachés, forment

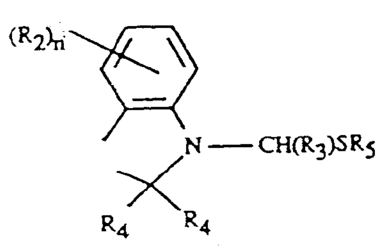 ou 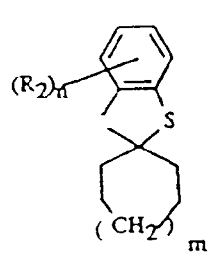 ,

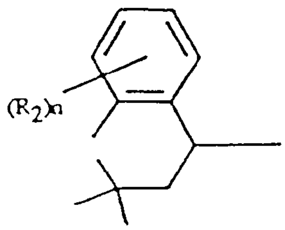 , 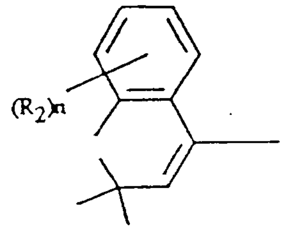 , ou 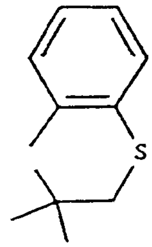 ;

$A_4$ est indépendamment H ou $-CH_2-S-R_1$, l'un au moins des $A_4$ étant $-CH_2-S-R_1$ ;
$A_5$ est H, un groupe alkyle en $C_1-C_{16}$, aryle en $C_6-C_{12}$ ou $-CH_2-S-R_1$ ;
$A_6$ est H ou un groupe alkyle en $C_1-C_{25}$ ;
m est 0, 1 ou 2 ;
n est 1, 2, 3 ou 4 ;
p est 1 à 20 ;
$L_1$ est une liaison directe, un groupe alkylène en $C_2-C_{12}$ linéaire ou ramifié, arylène en $C_6-C_{12}$, ou

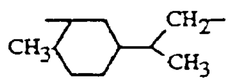 ;

et

$L_2$ est un groupe alkylène en $C_2-C_{12}$, arylène en $C_6-C_{12}$, xylylène, alkylène en $C_4-C_{12}$ qui est interrompu par $-N(CH(R_3)-S-R_5)$ où $R_5$ est tel que défini ci-dessus, ou bien $L_2$ est

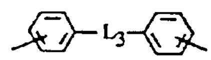

où $L_3$ est une liaison directe, un groupe alkylène en $C_1-C_6$, S, -NH- ou O.

**15.** Composé selon la revendication 14, dans lequel $L_2$ est un groupe phénylène ou

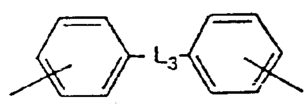

où $L_3$ est un groupe alkylène en $C_1-C_6$, S, -NH-, O ou une liaison directe.

**16.** Procédé pour stabiliser une matière organique sujette à une dégradation provoquée par la lumière, la chaleur ou l'oxydation, qui consiste à incorporer à ladite matière au moins un composé de formule (A) à (H) tel que défini dans la revendication 1.